Europäisches Patentamt

European Patent Office

Office européen des brevets

(19)

(11) EP 0 533 719 B1

# (12) FASCICULE DE BREVET EUROPEEN

(45) Date de publication et mention
de la délivrance du brevet:
**29.01.1997 Bulletin 1997/05**

(21) Numéro de dépôt: **91910511.4**

(22) Date de dépôt: **30.05.1991**

(51) Int Cl.6: **G01N 33/53**, G01N 33/76

(86) Numéro de dépôt international:
**PCT/FR91/00427**

**WO 91/19195 (12.12.1991 Gazette 1991/28)**

(54) **METHODE DE DETECTION ET/OU DE DOSAGE DES HORMONES**

VERFAHREN FÜR DEN NACHWEIS UND/ODER DIE BESTIMMUNG VON HORMONEN

METHOD FOR DETECTING AND/OR ASSAYING HORMONES

(84) Etats contractants désignés:
**AT BE CH DE DK ES FR GB GR IT LI LU NL SE**

(30) Priorité: **01.06.1990 FR 9006863**

(43) Date de publication de la demande:
**31.03.1993 Bulletin 1993/13**

(73) Titulaires:
• **INSTITUT NATIONAL DE LA RECHERCHE
AGRONOMIQUE
F-75341 Paris Cédex 07 (FR)**
• **CENTRE NATIONAL DE
LA RECHERCHE SCIENTIFIQUE (CNRS)
75794 Paris Cédex 16 (FR)**

(72) Inventeur: **MAUREL, Marie-Christine
F-37100 Tours (FR)**

(74) Mandataire: **Orès, Bernard et al
Cabinet ORES
6, Avenue de Messine
75008 Paris (FR)**

(56) Documents cités:
**EP-A- 0 098 590        EP-A- 0 193 881
US-A- 3 991 174**

• **JOURNAL OF CLINICAL IMMUNOASSAY, vol.
12, no. 1, 1989, (US); P.E. GARRETT, pp. 18-20/**

Remarques:
Le dossier contient des informations techniques présentées postérieurement au dépôt de la demande et ne figurant pas dans le présent fascicule.

EP 0 533 719 B1

## Description

La présente invention est relative à une méthode de détection et/ou de dosage des hormones y compris les hormones placentaires.

On entend par hormones, les substances élaborées par les différentes glandes endocrines humaines et animales et notamment, les hormones hypophysaires (FSH, LH, TSH, GH, ACTH, prolactine, ocytocine, MSH, ADH), les hormones placentaires apparentées [choriogonadotropines humaines (hCG) et équines (eCG), choriosomatomammotropine (CS), GH placentaire], les hormones thyroïdiennes, les hormones surrénaliennes, les hormones gonadiques et les hormones pancréatiques.

Selon leur structure chimique, ces hormones appartiennent à différentes catégories : les hormones dérivées d'amino-acides (hormones thyroidiennes, hormones de la médullosurrénale), les hormones peptidiques et protidiques (hormones pancréatiques, GH, ACTH, prolactine, MSH, ADH, ocytocine), les hormones stéroidiennes (hormones gonadiques, hormones de la corticosurrénale) et les hormones glycoprotéiques (LH, FSH, TSH, hCG, eCG).

Les hormones glycoprotéiques sont caractérisées par une même organisation structurale : elles sont formées par l'association de deux sous-unités $\alpha$ et $\beta$. La sous-unité $\alpha$ est identique pour toutes les hormones glycoprotéiques d'une même espèce animale. A l'inverse, la sous-unité $\beta$ est différente d'une hormone à l'autre et confère la spécificité d'action. Les hormones peptidiques, de même, présentent une parenté structurale ainsi que les hormones polypeptidiques (exemple : GH et prolactine).

De manière générale, ces hormones ne sont pas sécrétées à des taux constants mais présentent au contraire des variations de concentration circulante de faible amplitude (micropulsatilité) ou, dans certains cas, de forte amplitude (ex : pic de sécrétion de la FSH puis de la LH en période préovulatoire). Il est donc nécessaire de disposer de méthodes de dosage qui permettent de mesurer de façon précise et spécifique le taux circulant de ces différentes hormones, dans le sang ou dans tout autre fluide biologique possible.

Ces méthodes de dosages doivent être, de plus, très sensibles, dans la mesure où les concentrations basales de ces différentes hormones sont relativement faibles (pour les hormones hypophysaires par exemple, de 0,1 ng/ml à quelques ng/ml), en dehors de tous cas pathologiques.

A titre d'exemple, la détection et/ou le dosage des hormones gonadotropes LH et FSH est particulièrement importante dans l'étude de la physiologie de la reproduction mâle et femelle chez les animaux et l'Homme. Chez les mammifères, par exemple, le fonctionnement de l'ovaire comporte pendant la période d'activité génitale (continue ou saisonnière selon les espèces) une succession de cycles ovariens au cours desquels les gamètes femelles mûrissent à intervalles réguliers.

Le cycle ovarien comporte deux phases : la phase folliculaire (croissance du follicule et maturation de l'ovocyte) aboutissant à l'ovulation, période propice à la fécondation, et la phase lutéale (formation du corps jaune). La FSH est responsable de la croissance et de la maturation du follicule préovulatoire ; la LH intervient plus particulièrement dans la croissance folliculaire terminale de ce dernier et dans le déclenchement de l'ovulation. En effet, l'ovulation intervient à un moment très précis et constant, pour chaque espèce, après le pic de LH (de 17 à 24 heures selon les espèces). Il apparaît donc utile et important de pouvoir disposer, aussi bien au laboratoire pour des dosages très précis que sur le terrain ou à domicile pour la détection du pic préovulatoire de LH (pour prévoir le meilleur moment pour une insémination artificielle, par exemple), d'outils de détection spécifiques de la LH, chez différents animaux et chez l'Homme.

De même, la possibilité de détecter des niveaux circulants très bas de la choriogonadotropine (CG) est particulièrement importante pour la mise au point de kits de diagnostics de gestation les plus précoces possibles, spécialement chez la femme.

Chez l'Homme, un certain nombre de tests réalisables à domicile à partir de l'urine ont été développés et commercialisés pour la LH et la hCG.

En ce qui concerne la LH, un certain nombre de kits sont maintenant disponibles et permettent de déterminer de façon précise la date de l'ovulation. Ces tests utilisent des anticorps monoclonaux et sont basés soit sur une réaction d'agglutination ("DISCRETEST", CHEFARO pour ORGANON ; "HI GONADO TEST", MOSHIDA) soit sur une réaction immunoenzymatique ("OVUSTICK", MONOCLONAL ANTIBODIES ; "OVUTEST", CLONATEC ; "FIRST REPONSE" ; "REVELATEST O", ARLEY-DIAGNOSTIC).

En ce qui concerne la hCG, plusieurs tests de grossesse sont disponibles et sont réalisés à partir d'anticorps polyclonaux ou, le plus fréquemment, d'anticorps monoclonaux. On peut distinguer selon leur principe :

- les tests d'hémagglutination (ou tests à anneaux) ("PRIMOTEST RAPIDE" de FUMOUZE ; "BETA-TEST 100 UI" de SOEKAMI-LEFRANCQ ; "G-TEST" de THERANOL ; "ELLE-TEST BETA" de DEGLAUDE) ;
- les tests d'agglutination de particules d'or ("PREDICTOR COLOR" de NICHOLAS) ;
- les tests imunoenzymatiques à lecture indirecte (apparition d'une couleur bleue sur bandelette : "BLUE TEST" de CLONATEC) ou à lecture directe [apparition d'un signe particulier (point, barre ou signe +) : "BLUE POINT" de CLONATEC ; "G-TEST LOGIC" de THERANOL ; "PREVISION" de PHARMYGIENE] ;

- les tests associant anticorps monoclonaux et migration sur membrane, sans enzyme de révélation : "CLEAR BLUE EVIDENCE" de POLIVE-TRICOSTERIL ; "PRIMOTEST MINUTE" de FUMOUZE ; "G-TEST PRO" de THERANOL ; "FIRST RESPONSE" de TALCO ; "TEST PACK PLUS hCG" de ABBOTT). Ils sont caractérisés par l'apparition d'un signe coloré (bandes roses, barre bleue, etc...).

De manière générale, les méthodes de dosages, actuellement utilisées en clinique humaine, pour ces différentes hormones sont d'une part des méthodes radioimmunologiques (par exemple : système "AMERLEX-M" d'AMERSHAM, système "MAIAclone" de SERONO pour hLH, hFSH, hTSH, hCG, etc...) et d'autre part, des méthodes enzymo-immunométriques ou immunométriques (utilisant un marqueur non-enzymatique : particules de latex, chélate d'europium). Parmi les systèmes enzymo-immunométriques, certains utilisent un signal fluorescent (système enzymo-microparticulaire "IMX" de ABBOTT ; "STRATUS SYSTEM" de AMERICAN DADE pour LH, FSH, hCG...) ou luminescent (système "AMERLITE" d'AMERSHAM pour LH, TSH, FSH, hCG ; système "MAGIC LITE" de CIBA CORNING pour TSH, prolactine...). Parmi les systèmes immunométriques, on peut citer notamment le procédé de dosage "DELFIA" de PHARMACIA utilisant l'europium comme marqueur fluorescent, pour hLH, hFSH, hTSH, hCG, etc...

Chez les animaux, les méthodes de dosage de la LH sont notamment :

- le dosage de la LH dans l'urine chez le Gorille (N.M. CZEKALA et al., J. Reprod. Fert., 1988, 82, 255-261), qui utilise deux anticorps monoclonaux dont l'un est fixé sur une plaque de microtitration et dont l'autre est couplé à de la phosphatase alcaline. Le temps d'incubation total de ce test est de 4 heures (2 h : incubation de l'échantillon sur l'anticorps fixé ; 1 h : incubation du deuxième anticorps couplé ; 30 à 50mn : révélation enzymatique). La sensibilité de ce dosage est de 0,5 ng/ml.
- le dosage de la LH bovine plasmatique (sérum ou milieu de culture), par une méthode radioimmunologique (R. HOIER et al., Theriogenology., 1988, 30, 235-243). Ce dosage est réalisé en présence de deux anticorps (AC$_1$ : anti-LH fait chez le lapin, AC$_2$ : anti-IgG de lapin) et de LH radioactive (marquée à l'iode 125).

Il s'agit d'une méthode par compétition, le premier anticorps (AC$_1$) se liant à la LH de l'échantillon ou à la LH* radioactive ajoutée (incubation de 36 à 48 h). La séparation du complexe anticorps-LH, de la LH* libre est réalisée en utilisant le deuxième anticorps (AC$_2$) immobilisé sur les parois d'un tube (incubation : 3 heures, sous agitation à 37°C). La sensibilité de cette méthode est de l'ordre de 1,5 ng/ml.

- un dosage ELISA de la LH de souris, de rat, d'ovins et de bovins, applicable au sérum, aux milieux de culture de tissus et aux tampons, (J.L. SPEAROW et al., Biol. Reprod., 1987, 37, 595-605), qui utilise le principe de la compétition entre une LH couplée à de la peroxydase et la LH de l'échantillon, vis-à-vis d'un anticorps (AC$_1$) qui est soit un immunsérum anti-LH ovine fait chez le lapin, soit un anticorps monoclonal anti-LHβ bovine, soit un immunsérum anti-LHβ ovine fait chez le poulet.

Pour l'anticorps préparé chez le lapin, l'incubation AC1-échantillon est de 16 à 24 h à 20°C sous agitation ; l'ajout de la LH-peroxydase nécessite une nouvelle incubation de 16 à 24 h à 4°C. La révélation est réalisée à l'aide de TMB (30 mn à 2 h).

Dans ces conditions, la sensibilité de la méthode est de 79 pg/ml.

- une méthode de dosage ELISA de la LH bovine plasmatique (W.G. ABDUL-AHAD et al., J. Reprod. Fert., 1987, 80, 1-9). Il s'agit d'une méthode sandwich sur plaques de microtitration. Le premier anticorps (AC$_1$) est une IgG anti-LH bovine de lapin et le deuxième anticorps (AC$_2$) est le fragment Fab' préparé à partir du premier anticorps, conjugué à la peroxydase.

Ils présentent un protocole court (4 heures) et un protocole long (20 heures). Le seuil de détection obtenu avec le protocole court est de 260 pg/ml et celui obtenu avec le protocole long est de 70 pg/ml.

La révélation de la peroxydase se fait avec l'OPD.

Une autre publication des mêmes Auteurs (620th Meeting Dublin, Biochem. Soc. Transactions, 1985, 15, 277-278) relate le même procédé de dosage, utilisant comme AC$_2$ un conjugué Fab'-β lactamase.

Dans cet Article, il est spécifié que la durée du dosage est de 4 h 30 et que le seuil de détection est de 420 pg/ml.

- il faut également citer le dosage de la LH chez le singe Rhésus par un RIA qui utilise de la LH ovine marquée et un anti-sérum anti-LH ovine qui réagit avec les LH de nombreuses espèces.

Les méthodes de dosage de la FSH chez les animaux sont de type radioimmunologique essentiellement, de même pour les autres hormones.

Un dosage immunoenzymatique de la prolactine de rat a été décrit (A.P. SIGNORELLA et al., Anal. Biochem., 1984, 136, 372-381) . Ce dosage, de type compétitif, donne un seuil de détection fixé à 0,6 ng/ml en appliquant un protocole long (24 heures).

Cependant, de manière générale, pour le dosage des hormones, tant chez l'Homme que chez l'animal, malgré la diversité des méthodes de dosages proposées et appliquées, de nombreux travaux mentionnent le problème d'interférences non-spécifiques dans les dosages, entraînant l'obtention de résultats erronés (cas des "faux positifs" ou des "faux négatifs") [J.P. GOSLING, Clin. Chem., 1990, 36, 1408-1427 ; K.A. BRENSING, Horm. Metabol. Res., 1989, 21, 697-698 ; P.E. GARRETT, J. Clin. Immunoassay, 1989, 12, 18-19] . Ce problème d'interférences non-spécifiques conduit à une perte dans la sensibilité et le seuil de détection du dosage. On peut notamment citer le cas d'un dosage immunoenzymatique de hCG (B. LONGHI et al., 1986, J. Immun. Meth., 92, 89-95) pour lequel le seuil de détection a été évalué à 2,2 UI/l dans un milieu sans sérum et à 10,4 UI/l dans un milieu contenant 20 % de sérum humain "hCG négatif".

Un certain nombre de solutions à ce problème d'interférence, ont été proposées, mais celles-ci aboutissent dans tous les cas, à une perte de sensibilité du dosage. Il s'agit notamment :

- de supprimer la partie Fc du deuxième anticorps et de coupler directement l'enzyme sur un fragment Fab' [W.G. ABDUL-AHAD et al., J. Reprod. Fert., 1987, 80, 1-9]. Il faut, dans ce cas, appliquer des temps d'incubation et de révélation enzymatique plus longs pour pallier la perte de sensibilité due au traitement du deuxième anticorps : un seuil de détection de 70 pg/ml pour la LH bovine exige un protocole de 20 heures, un protocole court de 4 heures ne donnant qu'un seuil de détection à 260 pg/ml.
- d'appliquer un test permettant de mesurer le niveau des signaux non-spécifiques dépendants de l'échantillon ; pour cela, l'anticorps spécifique nonmarqué ($AC_1$) est substitué par un autre anticorps similaire à $AC_1$ mais d'une toute autre spécificité (P. KASPAR et al., J. Immun. Meth., 1988, 108, 61-69). Ceci permet de déterminer la valeur du signal non-spécifique, intrinsèque à chaque échantillon, et d'en tenir compte pour la calibration du dosage mais cela ne supprime en rien ce problème.
- de saturer l'anticorps spécifique avec de l'avidine, dans le cas d'un dosage ELISA de l'oestradiol-17β, de type compétitif utilisant le complexe avidine-biotine (D.M. BODMER et L.X. TIEFENAUER, 1990, J. Immunoassay, 11, 139-145).

Ainsi, l'ensemble des méthodes précitées présentent un certain nombre d'inconvénients :

. des temps d'incubation longs, notamment dans le cas des LHs animales, non adaptés au dosage ou à la détection sur le terrain (4 h à plus de 24 h) ;
. des dosages qui parfois, ne peuvent pas être réalisés sur le sang total ;
. l'absence de polyspécificité d'espèces de ces dosages ;
. l'existence de signaux non-spécifiques importants, lors de la lecture des résultats, qui peuvent notamment entraîner des difficultés d'interprétation de ces derniers ;
. un seuil de détection élevé, qui en résulte, variant de 100 pg à 1500 pg/ml dans le cas des LHs animales par exemple.

C'est pourquoi la Demanderesse s'est donné pour but de pourvoir à un procédé de dosage des hormones élaborées par les différentes glandes endocrines, applicables à de nombreuses espèces animales, y compris l'homme, qui répond mieux aux besoins de la pratique que les procédés de l'Art antérieur, notamment :

- en ce qu'il peut être réalisé en moins de 3 heures, selon un protocole simple dans des conditions qui peuvent être qualifiées de rustiques,
- en ce qu'il offre une lecture non-ambiguë du résultat, de type "tout ou rien", par l'obtention d'une couleur dans le cas de la détection d'un pic de sécrétion aiguë d'une hormone (exemple : pic préovulatoire de LH), alors que les tests de l'Art antérieur, lorsqu'ils sont basés sur une réaction colorimétrique, nécessitent une interprétation des résultats bien délicate pour une personne non-expérimentée (par exemple la distinction d'un bleu pâle ou foncé ou tout autre nuance de couleur, lesquelles nuances de couleur peuvent de plus, être directement dépendantes de la température, ce qui introduit un facteur supplémentaire d'erreur),
- en ce qu'il peut être semi-quantitatif sans besoin d'aucun appareil de lecture, si une gamme étalon de l'hormone à doser est déposée, en plus des échantillons à tester, notamment pour distinguer, pour un même animal, le prélèvement contenant, pour la LH par exemple, le début du pic de LH, celui contenant le sommet du pic (valeur maximale) et celui contenant la fin du pic (valeur descendante) ; dans le cas de la LH un tel procédé permet ainsi de synchroniser l'insémination artificielle par rapport au début du pic ou par rapport au sommet du pic et permet également de distinguer les pulses de sécrétion de la LH chez le mâle ou la femelle après une incubation du substrat de 15 à 30 mn si nécessaire, pour une étude de la micropulsatilité,

- en ce qu'il présente à la fois une polyspécificité d'espèce et une spécificité de substance,
- en ce qu'il est reproductible,
- en ce qu'il est réalisable sur le terrain (lecture à l'oeil nu) et
- en ce qu'il peut être parfaitement quantitatif en augmentant de façon importante la sensibilité du dosage et en abaissant le seuil de détection.

La présente invention a pour objet un procédé de détection et/ou de dosage immunologique d'hormones hypophysaires humaines ou animales, dans des milieux de culture ou dans des fluides biologiques, mettant en oeuvre au moins deux anticorps, spécifiques de l'hormone à doser, lequel procédé est caractérisé en ce que pour augmenter la sensibilité et la spécificité de ladite détection et/ou dudit dosage, lesdits anticorps sont, -préalablement à leur mise en oeuvre dans ladite détection ou ledit dosage-, préincubés dans un milieu contenant du plasma ou du sérum dépourvu de l'hormone à détecter et/ou à doser, obtenu par hypophysectomie (milieu INC).

On entend par anticorps préincubé, au sens de la présente invention, un anticorps "épuisé", -par mise en contact ou passage sur colonne de chromatographie convenable-, par toute substance pouvant induire une interférence sérique non hormonale et non spécifique.

Conformément à l'invention, le fluide biologique est avantageusement du sérum, du plasma, du sang total, du lait ou de l'urine.

Dans la suite de la présente Demande, le milieu contenant du sérum ou du plasma, dépourvu de l'hormone à détecter et/ou à doser est dénommé milieu INC.

Selon un mode de mise en oeuvre avantageux de ce procédé, ledit milieu INC est associé à un tampon à pH neutre et à un agent surfactant.

Selon un autre mode de mise en oeuvre avantageux de ce procédé, le milieu INC comprend un sérum ou un plasma dépourvu d'hormones hypophysaires, obtenu par hypophysectomie d'un animal et un tampon à pH neutre associé à un agent surfactant.

On peut notamment citer comme tampon approprié un tampon à pH neutre associé à un agent surfactant et notamment un tampon PBS-Tween-BSA.

Selon une disposition avantageuse de ce mode de mise en oeuvre, le milieu INC comprend un sérum ou un plasma de bélier hypophysectomisé et un tampon à pH neutre, associé à un agent surfactant.

Selon un autre mode de mise en oeuvre avantageux de ce procédé, le plasma ou sérum dépourvu de l'hormone à doser et le tampon sont dans un rapport 1:1.

Conformément à l'invention, ledit procédé est avantageusement un test enzymo-immunométrique, hautement spécifique et sensible, de détection d'au moins une hormone.

Selon un mode de mise en oeuvre avantageux dudit test enzymo-immunométrique, le premier anticorps, fixé sur un support solide, saturé en milieu INC et le deuxième anticorps, éventuellement couplé à une enzyme appropriée, préincubé dans un milieu INC, sont mis en contact avec le fluide biologique à tester, après quoi l'activité enzymatique liée à la phase solide et/ou libre est révélée par tout moyen approprié.

Selon une disposition avantageuse de ce mode de mise en oeuvre, lorsque le deuxième anticorps préincubé dans ledit milieu INC n'est pas couplé à une enzyme, la révélation de la réaction est alors réalisée par l'introduction d'un troisième anticorps couplé à une enzyme appropriée, préincubé dans ledit milieu INC et se liant spécifiquement au deuxième anticorps.

Conformément à cette disposition, les premier et deuxième anticorps sont avantageusement choisis dans le groupe qui comprend les anticorps monoclonaux anti-FSH, anti-LH, anti-TSH, anti-GH, anti-ACTH, anti-prolactine, anti-ocytocine, anti-ADH, anti-MSH et les anticorps polyclonaux anti-FSH, anti-LH, anti-TSH, anti-GH, anti-ACTH, anti-prolactine, anti-ocytocine, anti-ADH et anti-MSH.

Selon une modalité avantageuse de cette disposition, les anticorps polyclonaux anti-LH sont notamment obtenus par immunisation d'un animal approprié à l'aide d'un antigène comprenant un mélange équimolaire de différentes fractions purifiées de LH ovine répertoriées 1051, 1055, 1072, 1063, 1085 et 1086, puis par purification de l'immunsérum obtenu par chromatographie d'échange d'ions ou par chromatographie d'affinité, lesquels anticorps polyclonaux présentent un pourcentage de réactions croisées avec les autres hormones glycoprotéiques telles que la FSH, inférieur à 4 %, c'est-à-dire une spécificité vis-a-vis de la LH, en ce qu'ils présentent une polyspécificité de reconnaissance de la LH de nombreuses espèces animales et notamment au moins des espèces ovines, bovines, caprines, porcines, canines, camelines, murines ainsi que les cervidés et en ce qu'ils présentent une affinité vis-à-vis de la LH ovine de l'ordre du $10^9 M^{-1}$, respectivement dans deux espèces animales différentes.

Conformément à cette modalité avantageuse, les premier et deuxième anticorps sont avantageusement choisis dans le groupe qui comprend des anticorps polyclonaux de Lapin anti-LH ovine et des anticorps polyclonaux de Cheval anti-LH ovine et le troisième anticorps est avantageusement choisi dans le groupe qui comprend les anti-IgG de Cheval et les anti-IgG de Lapin couplés à une enzyme appropriée.

Egalement conformément à cette modalité avantageuse, le premier anticorps est un anticorps polyclonal de Lapin anti-LH ovine, le deuxième anticorps est un anticorps polyclonal de Cheval anti-LH ovine et le troisième anticorps est un anti-IgG de Cheval couplé à une enzyme appropriée et notamment à une peroxydase ou à une β-galactosidase.

Les différentes isoformes de LH ovines sont de préférence en quantités équimolaires.

Une purification convenable desdits anticorps polyclonaux anti-LH peut être réalisée, selon le cas, soit par chromatographie d'affinité, soit par chromatographie d'échange d'ions.

De manière inattendue, la préincubation avec le milieu INC, tel que défini ci-dessus, permet :

- d'éliminer les problèmes de signal non-spécifique habituellement rencontrés dans ce type de dosage, en neutralisant toute interférence sérique non spécifique pouvant conduire à une diminution de la sensibilité d'un dosage,
- d'augmenter considérablement, de ce fait, la sensibilité de ce type de dosage, particulièrement importante dans le cas des hormones à faible taux circulant,
- de doser ainsi l'ensemble des hormones et notamment des hormones hypophysaires et placentaires appropriées, en obtenant des résultats correctement et aisément interprétables.

En conséquence, le dosage immunologique et plus particulièrement le dosage enzymo-immunométrique des hormones, conforme à l'invention, présente à la fois les caractéristiques d'un dosage quantitatif en apportant une très bonne sensibilité ainsi qu'une très bonne reproductibilité, et d'un dosage qualitatif par sa facilité de réalisation et d'interprétation.

L'efficacité et les avantages du procédé conforme à l'invention décrit ci-dessus est illustrée en particulier par le dosage enzymo-immunométrique de l'hormone lutéinisante (LH). En effet, ce dosage présente à la fois les caractéristiques d'un dosage quantitatif en apportant une très bonne sensibilité (seuil de détection : 60 pg/ml) ainsi qu'une très bonne reproductibilité, et d'un dosage qualitatif par sa facilité de réalisation et d'interprétation. Il peut donc être utilisé aussi bien au laboratoire pour des dosages très précis (ex : mesure de la micropulsatilité de la sécrétion de LH) que sur le terrain (détection du pic préovulatoire de LH pour l'insémination artificielle de femelles synchronisées et/ou de femelles superovulées, donneuses d'embryons).

Ce dosage de la LH, utilisable chez de nombreuses espèces : bovine, ovine, caprine, porcine, cameline, canine, murine, cervidés, permet la détection précise du pic préovulatoire de LH chez la femelle et présente un intérêt physiologique et agronomique évident. Il va, de plus, permettre une bonne estimation du moment optimal choisi pour l'insémination artificielle dans chaque espèce concernée. En effet, l'intervalle entre le pic de LH et l'ovulation est constant pour chaque espèce ; par contre, l'intervalle de temps séparant la venue en chaleurs (repère utilisé jusqu'à présent) et le pic de LH est très fluctuant selon les individus d'une même race.

Le test conforme à l'invention s'avère donc un outil indispensable pour bien maîtriser ce facteur de variabilité important pour la réussite de la fécondation des ovocytes et de la collecte d'embryons de bonne qualité. En effet, connaissant le moment précis de l'ovulation, grâce à la détection du pic de LH, et de l'insémination artificielle, on peut en déduire à quel stade de développement seront les embryons que l'on veut collecter. Ce point est important dans la mesure où des embryons trop jeunes ou trop avancés ne sont pas commercialisables (ex : avec membrane pellucide rompue).

Enfin, ce test de dosage de la LH animale est intéressant dans le cadre de la mise au point d'un nouveau traitement (synchronisation, superovulation) ou de l'extension de ces traitements à une race, voire à une espèce dont la physiologie précovulatoire est mal connue (ex : cervidés).

De même, le dosage enzymo-immunométrique de la LH humaine, réalisé selon le procédé conforme à convention, présente à la fois les caractéristiques d'un dosage quantitatif par la très bonne sensibilité acquise grâce audit procédé et d'un dosage qualitatif par sa facilité de réalisation et d'interprétation acquise également par le procédé selon l'invention. Il peut donc être utilisé en laboratoire ou à domicile pour un usage individuel ("Home test") ; il servira, dans ce dernier cas, de kit de détection du pic préovulatoire de LH, caractérisé par l'apparition d'une couleur verte non-ambiguë, et permettant de prévoir le moment de l'ovulation chez la femme.

Par ailleurs, lorsque l'on désire réaliser un dosage présentant une polyspécificité d'espèce, les anticorps doivent présenter des qualités particulières ; en ce cas, le choix des anticorps est important pour une mise en oeuvre appropriée du procédé conforme à l'invention. C'est dans ce but que la Demanderesse a également mis au point des anticorps particulièrement adaptés au procédé conforme à l'invention.

La présente invention a également pour objet un réactif pour la mise en oeuvre du procédé de détection et/ou de dosage immunologique d'hormones chez l'animal y compris l'homme, conforme à l'invention, caractérisé en ce qu'il comprend un anticorps anti-hormone propre à être utilisé comme deuxième et/ou troisième anticorps dans ledit procédé, lequel anticorps est préincubé dans un milieu INC.

La présente invention a également pour objet un réactif pour la mise en oeuvre du procédé de détection et/ou de dosage immunologique d'hormones chez l'animal y compris l'homme, conforme à l'invention, caractérisé en ce qu'il comprend un anticorps anti-hormone propre à être utilisé comme premier anticorps dans ledit procédé, lequel premier anticorps est fixé sur un support solide, saturé en milieu INC.

La présente invention a, de plus, pour objet une trousse de détection et/ou de diagnostic d'hormones hypophysaires (la FSH, la LH, la TSH, la GH, l'ACTH, la prolactine, l'ocytocine, l'ADH, la MSH) chez l'homme ou l'animal, ou kit pour la mise en oeuvre du test conforme à l'invention, caractérisé en ce qu'il comprend outre des quantités utiles de tampons pour la mise en oeuvre de ladite détection :

- un premier anticorps, spécifique de l'hormone à doser, choisi parmi les anticorps anti-hormone à doser, fixé sur un support solide, saturé en milieu INC conforme à l'invention ;
- un deuxième anticorps, spécifique de l'hormone à doser, choisi parmi les anticorps anti-hormone à doser, couplé à une enzyme appropriée et préincubé dans ledit milieu INC conforme à l'invention ;
- un substrat de révélation de l'enzyme et
- un milieu INC.

Selon un mode de réalisation avantageux dudit kit, lorsqu'il est mis en oeuvre pour le dosage de la LH, il comprend :

- un premier anticorps polyclonal, fixé sur un support solide, saturé en milieu INC, choisi parmi les anticorps polyclonaux de Lapin anti-LH ovine et les anticorps polyclonaux de Cheval anti-LH ovine ;
- un conjugué enzyme-anticorps, dans lequel l'anticorps est choisi dans le groupe qui comprend les anticorps polyclonaux de Lapin anti-LH ovine, les anticorps polyclonaux de Cheval anti-LH ovine, lesquels conjugués sont préincubés dans un milieu INC ;
- un substrat de révélation de l'enzyme ;
- un capillaire de prélèvement ; et
- un milieu INC.

Selon un autre mode de réalisation avantageux de ce kit, l'enzyme est choisie dans le groupe qui comprend les peroxydases et la β-galactosidase.

Les substrats de révélation de la peroxydase sont avantageusement l'OPD et l'ABTS ; le substrat de révélation de la β-galactosidase est avantageusement le 4-méthylumbelliféryl-β-D-galactopyranoside (MUG) et l'ortho-nitrophényl-β-O-galactopyranoside (ONPG).

Le support solide est avantageusement choisi parmi les supports connus tels que les plaques de microtitration, les sticks, les bandelettes, les billes ou les membranes.

Selon un autre mode de réalisation avantageux de ce kit, il comprend :

- une série de supports solides convenables revêtus d'un premier anticorps choisi parmi les anticorps anti-hormone à doser, lesquels supports solides sont saturés en milieu INC ;
- un deuxième anticorps choisi dans le groupe qui comprend les anticorps anti-FSH, anti-LH, anti-TSH, anti-GH, anti-ACTH, anti-prolactine, anti-ocytocine et anti-ADH, différents du premier anticorps, préincubés dans un milieu INC ;
- un conjugué peroxydase-anti-IgG, préincubé dans un milieu INC ;
- un substrat de révélation de la peroxydase ; et - un milieu INC.

Selon une disposition avantageuse de ce mode de réalisation, il comprend :

- une série de supports solides revêtus d'un premier anticorps choisi dans le groupe qui comprend les anticorps polyclonaux de Lapin anti-LH ovine, lesquels supports solides sont saturés en milieu INC ;
- un deuxième anticorps choisi dans le groupe qui comprend les anticorps polyclonaux de Cheval anti-LH ;
- un conjugué peroxydase-anti-IgG de Cheval préincubé dans un milieu INC ;
- -un substrat de révélation de la peroxydase
- un capillaire de prélèvement ; et
- un milieu INC.

Selon une autre disposition avantageuse de ce mode de réalisation, il comprend :

- une série de supports solides revêtus d'un premier anticorps choisi dans le groupe qui comprend les anticorps polyclonaux de Lapin anti-LH ovine, lesquels supports solides sont saturés en milieu INC ;
- un conjugué β-galactosidase-anticorps polyclonaux de Cheval anti-LH ovine préincubé dans un milieu INC ;
- un substrat de révélation de la β-galactosidase ;
- un capillaire de prélèvement ; et

- un milieu INC.

Selon une disposition avantageuse de ce mode de réalisation, il comprend :

- une série de supports solides convenables revêtus d'un anticorps choisi dans le groupe qui comprend les anticorps polyclonaux de Lapin anti-LH ovine conformes à l'invention et les réactifs conformes à l'invention contenant l'anticorps polyclonal de Lapin précité, lesquels supports solides sont éventuellement saturés en milieu INC ;
- des doses appropriées d'un anticorps choisi dans le groupe qui comprend les anticorps polyclonaux de Cheval anti-LH ovine et les réactifs conformes à l'invention contenant l'anticorps polyclonal de Cheval précité ;
- des doses appropriées de conjugués peroxydase-anti-IgG de Cheval préincubés dans un milieu INC, conformément au réactif décrit ci-dessus ;
- des quantités appropriées d'un substrat de révélation de la peroxydase ; et
- un capillaire de prélèvement.

Selon une autre disposition avantageuse de ce mode de réalisation, il comprend :

- une série de supports solides convenables revêtus d'un anticorps choisi dans le groupe qui comprend les anticorps polyclonaux de Lapin anti-LH ovine conformes à l'invention et les réactifs conformes à l'invention contenant l'anticorps polyclonal de Lapin précité, lesquels supports solides sont éventuellement saturés en milieu INC ;
- des doses appropriées de conjugués $\beta$-galactosidase-anticorps polyclonaux de Cheval anti-LH ovine préincubés dans un milieu INC, conformément au réactif décrit ci-dessus ;
- des quantités appropriées d'un substrat de révélation de la $\beta$-galactosidase ; et
- un capillaire de prélèvement.

Outre les dispositions qui précèdent, l'invention comprend encore d'autres dispositions qui ressortiront de la description qui va suivre.

L'invention sera mieux comprise à l'aide du complément de description qui va suivre, qui se réfère à des exemples de réalisation des anticorps polyclonaux conformes à l'invention et des exemples de mise en oeuvre du test conformes à l'invention.

Il doit être bien entendu, toutefois, que ces exemples et dessins sont donnés uniquement à titre d'illustration de l'objet de l'invention, dont ils ne constituent en aucune manière une limitation. **EXEMPE 1 : Préparation des anticorps polyclonaux anti-LHs animales**

1) Caractéristique de l'immunogène :

L'antigène utilisé pour les immunisations est un mélange équimolaire de différentes fractions purifiées de LH ovine (oLH). Ces fractions ont été répertoriées ainsi :

| oLH 1051 | 1083 |
|---|---|
| 1055 | 1085 |
| 1072 | 1086 |

2) Procédé d'obtention des anticorps polyclonaux anti-LH :

a.2) anticorps fait chez le cheval (poney $MW_5$) Chaque injection contient 1 mg du mélange oLH précisé ci-dessus, dissous dans 2,5 ml de sérum physiologique stérile et préparé en émulsion dans 2,5 ml d'adjuvant de Freund complet.

Le protocole d'immunisation consiste en 6 injections suivies de 8 rappels après chacun desquels a été réalisée une saignée, 2 semaines plus tard. La séquence exacte des injections est illustrée sur le schéma I ci-après :

```
injections-↑-rappels

  1 2 3 4 5  6   R₁      R₂      R₃      R₄      R₅      R₆      R₇      R₈
 ─────────────────────────────────────────────────────────────────────────
  Toutes les    1       2       3       2       5       7       2       1
  2 semaines   mois    mois    mois    mois    mois    mois    mois    mois
               1/2
  pas de    ↓  une saignée 2 semaines après chaque
  saignée      rappel
```

Schéma I

b.2) anticorps fait chez le lapin :

Chaque injection contient 0,1 mg du mélange oLH, dissous dans 0,5 ml de sérum physiologique stérile. Le tout est préparé en émulsion dans 0,5 ml d'adjuvant de Freund complet et injecté en multiples points le long de la colonne vertébrale (injections intra-dermiques).

Le protocole d'immunisation a consisté en 4 injections réalisées toutes les deux semaines. Puis, a suivi une série de 12 rappels effectués selon la séquence illustrée ci-après dans le schéma II :

```
 R₁      R₂      R₃      R₄      R₅      R₆      R₇      R₈      R₉      R₁₀     R₁₁     R₁₂
 ────────────────────────────────────────────────────────────────────────────────────────
   2 mois │3 mois │2mois  2mois  1mois │5 mois │      │2mois │2mois │2mois │1mois │
                                                4mois

 saignées :S₁      S₂                   S₃    S₄S₅    S₆     S₇        S₈    S₉    S₁₀
```

Schéma II

Les saignées sont réalisées deux semaines après les rappels indiqués à l'exception de $S_5$ qui a été réalisée quatre semaines après $R_7$.

Trois lapins ont été immunisés : deux ont reçu les 12 rappels, un n'a reçu que 6 rappels.

3) Procédés de purification des anticorps polyclonaux anti-LH :

a.3) Purification de l'immunsérum de cheval : Cette purification se fait par chromatographie d'échanges d'ions sur DEAE-Trisacryl (IBF-FRANCE) en colonne K26/40 de PHARMACIA.

L'immunsérum (15 ml) est dialysé une nuit contre un tampon Tris-HCl 0,025 M, NaCl 0,035 M, pH 8,8 à 4°C. Parallèlement, la colonne est équilibrée avec ce même tampon. Après filtration du sérum dialysé, celui-ci est passé sur la colonne avec un débit de 50 ml/heure. La fraction éluée obtenue à la sortie de colonne, contient les IgG du sérum et représente la fraction enrichie en anticorps que l'on utilise dans le dosage. Avant utilisation, cette fraction est dialysée contre du PBS pH 7,4 (0,01 M de $KH_2PO_4$/$K_2HPO_4$, NaCl 0,15 M). Elle est ensuite concentrée par ultrafiltration sur support MINICON (AMICON, Irlande) jusqu'à obtenir une concentration comprise entre 5 et 10 ng/ml. Elle est stockée sous forme d'aliquots à -20°C, diluée dans 50 % de glycérol bi-distillé.

b.3) Purification de l'immunsérum de lapin :

La purification se fait par chromatographie d'affinité sur protéine A. On utilise le gel Protéine A-Sépharose CL-4B (PHARMACIA) coulé dans une colonne 2 X 10 cm (PHARMACIA). Le sérum (5 ml) est préalablement dialysé contre du tampon phosphate 0,1 M pH 8, filtré et passé sur la colonne équilibrée avec le même tampon (débit : 15 ml/h).

Les immunoglobulines sont désorbées en utilisant un gradient de pH allant de 6 à 4,5 et préparé avec un tampon citrate-acide citrique 0,1 M puis avec un tampon pH 3 (acide acétique 0,1 N, NaCl 0,15 M).

Les fractions éluées sont neutralisées avec du tampon phosphate 1 M pH 8, et dialysées contre du PBS avant d'être concentrées par ultrafiltration (cf ci-dessus) et stockées à -20°C dans 50 % de glycérol bi-distillé.

Les protocoles expérimentaux utilisés en 3 a) et b) ont été réalisés selon les indications données par les fournisseurs et complétées avec "Techniques immunoenzymatiques" Th. TERNYNCK et S. AVRAMEAS, éditions INSERM, 1987.

Dans tous les cas, la concentration exacte des fractions purifiées est évaluée par une mesure de la densité optique à 280 nm en considérant qu'une d.o. de 1,4 correspond approximativement à une solution d'IgG à 1 mg/ml.

4) Caractéristiques et spécificité des anticorps anti-LH conformes à l'invention :

. réactions croisées :

Compte-tenu de la parenté structurale avec d'autres hormones glycoprotéiques, il est indispensable pour obtenir un dosage fiable, de disposer d'anticorps ne reconnaissant que la LH et ne présentant aucune réaction croisée avec la FSH et la TSH dans les espèces animales étudiées. En conséquence, diverses FSH et TSH ont été testées dans le dosage pour en évaluer le pourcentage de réaction croisée.

De la même façon, des LH de différentes espèces animales ont été testées dans le dosage et leur pourcentage de réaction croisée a été calculé.

Le pourcentage de réaction croisée est calculé de façon classique en comparant les courbes dose-réponse obtenues d'une part, avec une gamme de concentrations de chaque LH testée et, d'autre part, avec une gamme de concentrations de LH ovine. A partir de la courbe "référence" obtenue avec la LH ovine, soit Y la concentration donnant 50 % du signal optique maximal (E.D.50). A partir de la courbe obtenue avec une autre LH, soit X la concentration correspondante à la même densité optique que celle utilisée pour définir Y : Le pourcentage de réaction croisée est égal à $\frac{X}{Y}$ x 100

a) Réactions croisées obtenues avec les différentes LH testées :

| Origine de la LH | Bovine | Caprine | Porcine | Canine | Cameline | Murine (rat) |
|---|---|---|---|---|---|---|
| Pourcentage de réaction croisée | 97 % | 45,5 % | 34 % | 20 % | 55,6 % | 20 % |

b) Réactions croisées obtenues avec les FSH et TSH des espèces animales pour lesquelles la LH est reconnue par les deux anticorps (AP1 et AP2) spécifiques du dosage :

| Espèce animale | Ovine | Bovine | Porcine | Murine (rat) |
|---|---|---|---|---|
| FSH | 0,05 % | 3,12 % | 0,06 % | 0,03 % |
| TSH | 8 % | 2 % | 0,62 % | 1,15 % |

Il faut remarquer que le pourcentage de réaction croisée avec la TSH ovine est élevé (8 %) et est peut être dû à une contamination élevée dans la préparation hormonale utilisée.

. Affinité des anticorps anti-LH (anticorps lapin et cheval) utilisés dans le dosage :

La constante d'affinité (Ka) a été mesurée selon la méthode décrite par B. FRIGUET et al. ((1985), J. Immunol. Methods, vol. 77, 305-319) et est calculée selon la représentation de KLOTZ :

. anticorps de Lapin anti-LH ovine;

$$Ka = 1,87.10^9 M^{-1}$$

. anticorps de Cheval anti-LH ovine,

$$Ka = 2.10^9 M^{-1}.$$

**EXEMPLE 2 : Test immunoenzymométrique conforme à l'invention : dosage de LHs animales.**

A - Principe du dosage :

Il s'agit d'un dosage ELISA (Enzyme-Linked-ImmunoSorbant-Assay) c'est-à-dire que l'ensemble des réactifs sont liés à une phase solide. La phase solide utilisée est, de manière non limitative, une plaque de microtitration à 96 puits en Luxlon (plastique hydrophobe), mais le dosage peut être réalisé sur d'autres types de plaques ou d'autres types de supports, notamment des sticks, des bandelettes, des billes ou des membranes.

Le dosage conforme à l'invention, de type "sandwich", utilise deux anticorps polyclonaux tels que définis ci-dessus, c'est-à-dire fabriqués à partir d'un même antigène, la LH ovine, mais produits chez deux espèces animales différentes (le lapin et le poney) :

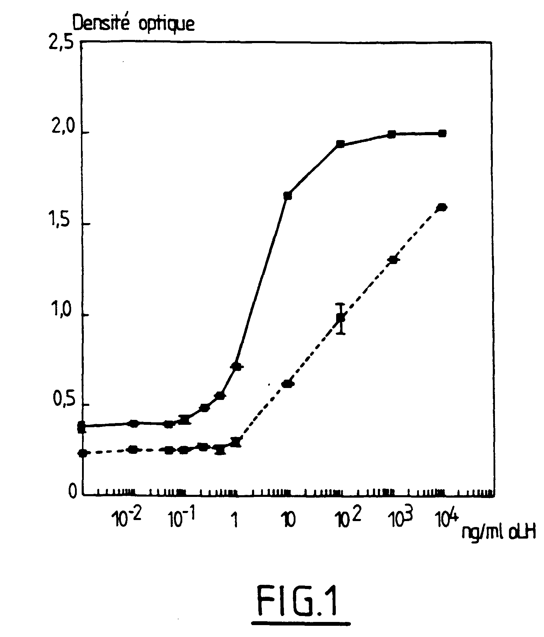

- le premier anticorps (AP1) est fixé sur le fond des puits d'une plaque de microtitration. Ce premier anticorps a été produit chez un lapin ;
- après lavages de la plaque, on sature éventuellement la plaque avec un milieu approprié additionné de sérum ou de plasma dépourvu de LH. Ce milieu est ci-après dénommé milieu INC ;
- l'échantillon à doser est incubé ; un volume de 10 µl par puits est nécessaire c'est-à-dire que le dosage peut se faire à partir d'une goutte de plasma ou de sang. Le dosage à partir du sang se fait dans un milieu modifié contenant de l'héparine, ci-après dénommé milieu S ;
- lavages de la plaque, incubation du deuxième anticorps préalablement dilué dans le milieu INC. Ce deuxième anticorps a été produit chez un poney ;
- lavages de la plaque, incubation d'un troisième anticorps couplé à une enzyme (anticorps de chèvre-anti-IgG de cheval couplé à la peroxydase ou à la β-galactosidase). Avant son dépôt, l'anticorps est préalablement dilué dans le milieu INC ;
- après lavages, l'activité enzymatique liée à la phase solide est révélée à l'aide d'un substrat chromogène ou fluorogène spécifique de l'enzyme utilisée. L'intensité de la réaction colorée ou du signal fluorescent obtenus est proportionnelle à la concentration de la LH contenue dans l'échantillon dosé comme le montrent les courbes de la figure 1. Le temps de réaction nécessaire est de 5 minutes pour une détection de pic préovulatoire et de 15 minutes pour une mesure quantitative précise de taux circulants bas.

Le rôle de l'ordre d'utilisation des deux anticorps spécifiques a été déterminé en comparant les deux combinaisons possibles :

. courbe (-■-) : AP1 Lapin
        AP2 Cheval et
. courbe (--■--) : AP1 Cheval
        AP2 Lapin.

Les résultats, présentés sur la figure 1, ont été obtenus à partir d'une gamme de dilutions de la LH ovine réalisée dans du tampon phosphate (gamme standard). Comme on le distingue très nettement, la combinaison AP1 Lapin/AP$_2$ Cheval (-■-) donne de meilleurs résultats tant d'un point de vue seuil de détection que sensibilité du dosage.

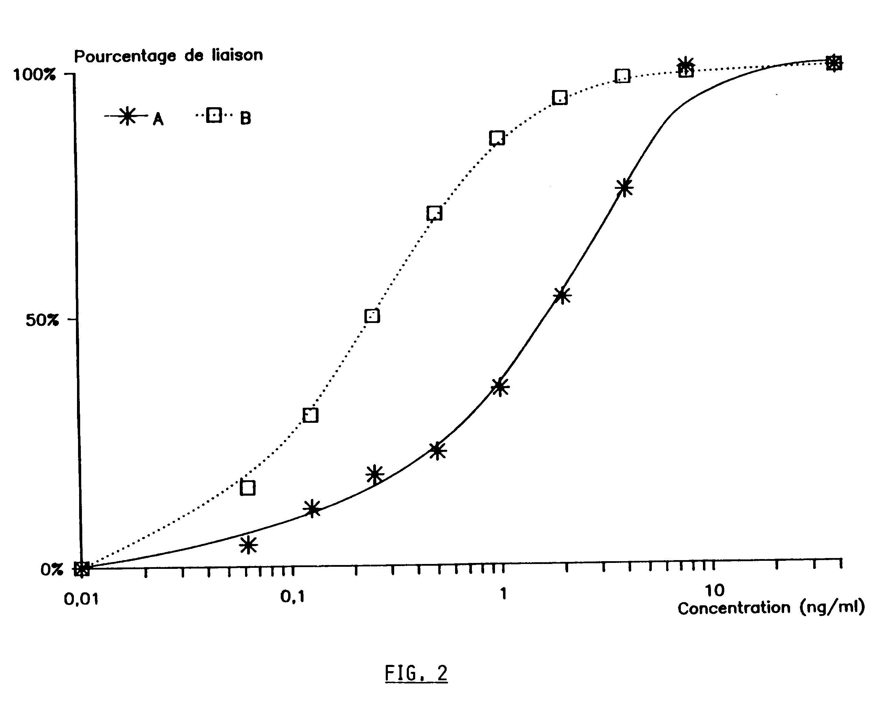

Le fait d'avoir utilisé deux anticorps faits chez deux espèces animales distinctes est crucial pour l'obtention de la sensibilité améliorée du test conforme à l'invention, par rapport aux tests de l'Art antérieur. Cette sélection particulière d'anticorps polyclonaux donne plus de probabilités à la molécule de LH d'être reconnue par des épitopes différents par chacun des immunsérums par rapport à des immunsérums faits chez la même espèce. La figure 2 montre la diminution de la sensibilité du dosage lorsqu'on utilise deux anticorps (AP1 et AP2) faits chez la même espèce (Cheval, par exemple : AP1 = AP Cheval à 10 µg/ml et AP2 = AP Cheval-β-galactosidase à 5 µg/ml).

L'utilisation d'un même anticorps comme AP1 et comme AP2 (courbe -*-) aboutit à une perte considérable de la sensibilité du dosage comme en témoignent les courbes de la figure 2. On observe, pour la concentration de oLH donnant 50 % de liaison, une sensibilité de 250 pg/ml dans le cas d'un protocole conforme à l'invention (AP1 Lapin, AP2 Cheval, AP3 anti Cheval-peroxydase ; courbe (..□..) contre 1,8 ng/ml dans le cas d'un protocole à deux anticorps identiques (courbe -*-), comme précisés ci-dessus. Ce dernier chiffre correspond à une efficacité résiduelle de 15 %, par rapport au dosage réalisé dans les conditions optimales (conformes à l'invention, voir notamment exemple 3 ci-après). Le pourcentage de liaison a été calculé selon la formule B-NS/T, dans laquelle B est la densité optique mesurée pour chaque point de la gamme, NS est la densité optique mesurée pour le point zéro de la gamme et T représente la densité optique mesurée pour le point le plus haut de la gamme.

B - Caractéristiques des milieux d'incubation conformes à l'invention :

1) Milieu de dilution du sang :

Dans le cas d'un dosage réalisé directement à partir du sang, le milieu de dilution du prélèvement doit être additionné d'héparine pour éviter la coagulation. Le sang est donc récupéré dans du PBS-Tween-BSA additionné de 0,2 % d'héparine, soit 100 μl d'héparine pour 50 ml de PBS-Tween-BSA (voir Exemple 3).

2) Milieu INC :

2.a) composition du milieu INC :

Ce milieu est composé d'au moins 50 % de sérum ou de plasma de bélier hypophysectomisé dilué dans du PBS-Tween-BSA.

2.b) obtention du sérum de bélier hypophysectomisé :

L'animal est mis à jeun 48 heures avant l'opération. Celle-ci est réalisée sous anesthésie ; l'ablation de l'hypophyse se fait par le plancher buccal.

Des prélèvements de sang sont effectués tous les jours afin de contrôler la chute du taux de LH circulante à partir de l'hypophysectomie. Lorsque celui-ci n'est plus détectable, l'animal est anesthésié et saigné à blanc. Le sang est collecté sous héparine pour récupérer le plasma ou est mis à coaguler pour en séparer le sérum. Plasma et sérum sont conservés à -20°C.

L'animal est saigné entre 5 et 7 jours après l'hypophysectomie.

2.c) Intérêt du milieu INC dans le dosage :

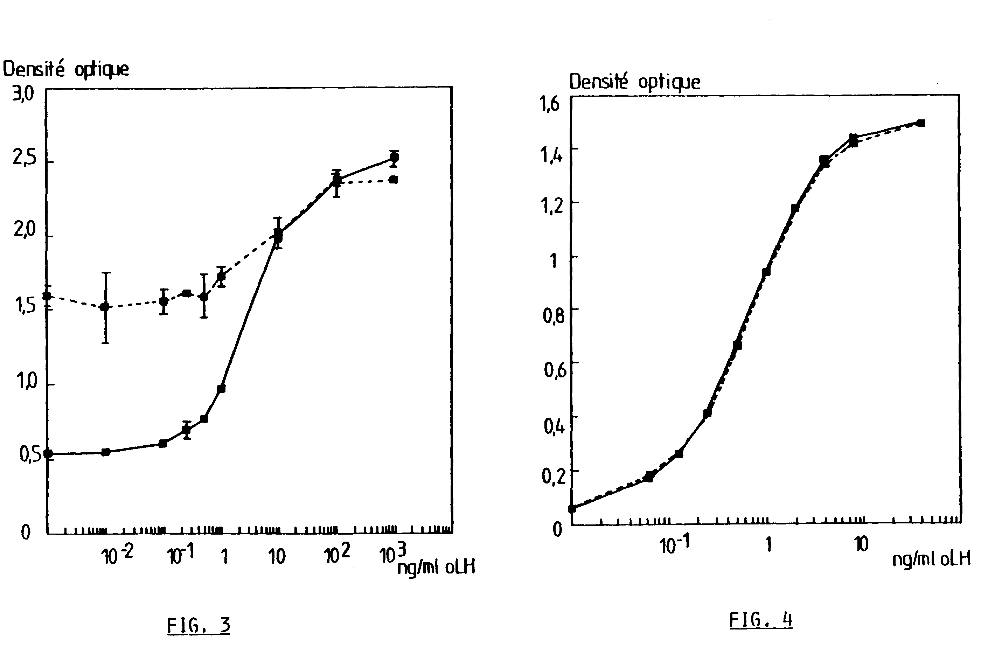

Il est indispensable, dans un dosage plasmatique, de réaliser la gamme standard dans un milieu de dilution contenant la même proportion de plasma que les préparations d'échantillons à doser. Si ceux-ci sont dilués dix fois, la gamme standard sera réalisée avec du sérum d'animal hypophysectomisé (LH$^+$) dilué dix fois dans du tampon de dilution normal (PBS-BSA-Tween). La figure 3 rapporte les résultats obtenus avec une gamme standard préparée dans du tampon de dilution (-■-) et ceux obtenus avec une gamme réalisée dans ce même tampon additionné de 10 % de sérum d'animal hypophysectomisé (--■--) (dosage de type AP3-peroxydase/OPD, voir Exemple 3). On observe une interférence, particulièrement gênante, du sérum LH$^-$ dans le dosage, surtout dans la zone des faibles concentrations de LH ovine (oLH), qui masque toute la sensibilité réelle du dosage.

En conséquence, les deux immunsérums anti-LH ont été épuisés sur des IgG de mouton afin d'éliminer toute interférence entre IgG provenant d'espèces animales différentes (ovine d'une part, lapine et équine d'autre part). Les résultats obtenus n'ont montré qu'une faible diminution du signal non-spécifique important, observé sans épuisement préalable.

Il semblerait qu'une autre source d'interférence puisse intervenir, due par exemple à une substance immunologiquement proche de la LH et reconnue comme telle.

Des résultats semblables ont été décrits dans un dosage radioimmunologique de la LH chez le singe Rhésus (NEILL et al., 1977, PECKHAM et al., 1977). En utilisant de la LH ovine comme traceur iodé et un anticorps anti-LH ovine, les Auteurs ont observé une interférence non spécifique très importante pour les faibles concentrations de gamme ; à l'inverse ils n'observaient pas d'interférence en utilisant de la LH de singe iodée et un anticorps anti-LH de singe. Cette substance "LH-like" n'a pas été purifiée et son effet interférant n'a pas été contourné par les Auteurs.

Le procédé conforme à l'invention a pour sa part, l'avantage de permettre d'éliminer cette difficulté, grâce au milieu INC qui a permis la mise au point d'un dosage très sensible, fiable et rapide.

C - Substrats et conjugués enzymatiques :

Ce dosage n'est pas dépendant de l'enzyme utilisée comme marqueur. Deux enzymes ont été utilisées jusqu'à présent, la peroxydase et la β-galactosidase, la première avec deux substrats chromogènes, la deuxième avec un substrat fluorogène.

1) Substrats utilisés :

\* pour la peroxydase :

. l'OPD (ortho-phénylènediamine)
. l'ABTS (2-2'-azino-bis(3-éthylbenzthiazoline-6-sulfonique acide).

- l'OPD est préparé extemporanément à 0,5 mg/ml dans du tampon acétate (0,1 M d'acétate de sodium) pH 5,6, additionné d'eau oxygénée à 0,2 %.

La mesure de la densité optique se fait à 492 nm ; la réaction se traduit par l'apparition d'une couleur orange à brune et est arrêtée avec 50 μl d'acide sulfurique 2N.

- l'ABTS est préparé dans du tampon citrate 0,05 M pH 4 : pour 12 ml de tampon citrate on rajoute 60μl d'ABTS stock et 50μl d'eau oxygénée à 2 %, la mesure de la densité optique se fait à 405 nm.

Sous l'action de la peroxydase, l'ABTS incolore devient vert.

. Tampon citrate : 2 volumes d'acide citrique ((5,3 g/l) $C_6H_8O_7$, $1H_2O$) + un volume de phosphate de sodium ($Na_2HPO_4$, 12 $H_2O$) (17,73 g/l). La réaction peut être arrêtée avec 20 μl de SDS 10 %, ceci n'ayant aucun caractère obligatoire.

* Pour la β-galactosidase :

- le 4-méthylumbelliféryl-β-D-galactopyranoside (MUG), produit commercial (SIGMA M 1633) :
2,5 mg sont dissous dans 12,5 ml de tampon phosphate 0,1 M, pH 7,4 (voir composition dans Exemple 3). La réaction est arrêtée avec 50 μl de carbonate de sodium 2 M.
La lecture du signal fluorescence se fait avec un spectrofluorimètre pour plaques ELISA.
La longueur d'onde d'excitation = 360 nm, la longueur d'onde d'émission = 448 nm.

2) Conjugués enzymatiques :

* l'anticorps conjugué à la peroxydase (AP3 : anticorps-anti-IgG de cheval couplé à la peroxydase est disponible dans le commerce : Jackson Immunoresearch Laboratories Inc., code n° 108-035-003.
* l'anticorps conjugué à la β-galactosidase a été réalisé selon deux optiques :

- soit l'anticorps anti-LH de cheval est couplé directement à la β-galactosidase (AP2-β-galactosidase),
- soit un anticorps anti-IgG de cheval est couplé à la β-galactosidase (AP3-β-galactosidase).

* réalisation du couplage :
Elle a été conduite selon le procédé de couplage au glutaraldéhyde en deux temps (Techniques Immuno-noenzymatiques, Th. TERNYNCK et S. AVRAMEAS, Editions INSERM, 1987, pages 33-34).
L'anticorps anti-LH de cheval est purifié sur chromatographie d'échanges d'ions (DEAE) ainsi que l'anticorps anti-IgG de cheval (fait chez le mouton).

D - Tampons :

* Tampon carbonate 0,1M pH 9,6 : diluer 10 fois une solution molaire de carbonate-bicarbonate de sodium dans de l'eau distillée.
* PBS diluer 100 fois une solution molaire de phosphate de potassium mono- et bi-potassique pH 7,4 dans une solution à 0,15 M de NaCl.
* PBS-Tween : ajouter 0,1 % (1 ml dans 1 litre) de Tween 20 dans le PBS.
* PBS-Tween-BSA : ajouter 0,2 % de BSA dans le PBS-Tween.

**EXEMPLE 3 : Dosage colorimétrique quantitatif avec le système AP3-peroxydase : dosage de LHS animales**

1.a) Système AP3-peroxydase/substrat ABTS :

* sensibilisation des plaques ou coating :
100 μl de l'anticorps anti-LH ovine de lapin (AP1) préparé dans du tampon carbonate 0,1 M pH 9,6 à la concentration de 7,5 μg/ml sont distribués par puits. La plaque est incubée 1 heure à 37°C puis une nuit à 4°C. Ensuite, la plaque est lavée avec du PBS-Tween 5 fois à raison de 300 μl par puits, soit manuellement, soit avec un laveur automatique pour plaque ELISA.
L'AP1 peut être préparé à une concentration différente : 10 μg/ml ou 5 μg/ml ; les concentrations extrêmes étant de 20 μg/ml et de 2,5 μg/ml.
* Surcoating ou saturation des puits :
Après lavage, les puits sont remplis de 100 μl de milieu INC et la plaque est incubée 30 minutes à 37°C. A cette étape, la plaque est soit utilisée pour la suite du dosage, soit vidée, mise à sécher à 37°C puis gardée dans une atmosphère sèche, scellée sous plastique.

EP 0 533 719 B1

* incubation des échantillons et de la gamme :

La gamme de oLH (LH ovine) est préparée dans du PBS-Tween-BSA contenant du sérum d'animal hypophysectomisé dilué au 1/10e. Elle comprend les concentrations suivantes : 60 pg/ml, 125 pg/ml, 250 pg/ml, 500 pg/ml, 1 ng/ml, 2 ng/ml, 4 ng/ml, 8 ng/ml et éventuellement 40 ng/ml de oLH. Elle est déposée en double à raison de 100 µl par puits.

Les plasmas à doser sont dilués 10 fois dans du PBS-Tween-BSA. Ils sont déposés en double ou en triple, à raison de 100 µl par puits. (La dilution de 1/10e n'est pas impérative et pourra être supérieure ou inférieure si cela est nécessaire). Le tout est mis à incuber 1 heure à 37°C. Après quoi, la plaque est lavée 5 fois avec du PBS -Tween (300 µl/puits) de la même façon que ci-dessus.

* incubation du deuxième anticorps anti-LH (cheval) :

Cet anticorps, AP2, est préalablement préparé dans du milieu INC : il est dilué à la concentration de 5 µg/ml dans le milieu INC et mis à incuber 30 minutes à 37°C avant d'être déposé dans les puits à raison de 100 µl par puits.

L'AP2 une fois distribué, la plaque est mise à incuber 1 heure à 37°C. Comme dans le cas de l'AP1 de lapin, la concentration de 5 µg/ml pour l'AP2 n'est pas obligatoire mais elle doit dans tous les cas être inférieure à celle de l'API. Elle peut être comprise entre 15 µg/ml (si AP1 est à 20 µg/ml) à 2,5 µg/ml, voire 1 µg/ml (si AP1 est à 2,5 µg/ml). Après l'incubation, la plaque est lavée 5 fois de la même façon que précédemment avec du PBS-Tween.

* incubation du troisième anticorps anti-IgG de cheval couplé à la peroxydase :

L'AP3 est également préincubé 30 mn à 37°C dans le milieu INC à la dilution de 1/5000e (selon l'indication du fabricant) avant d'être déposé dans les puits (100 µl par puits). On laisse incuber 1 heure à 37°C. La plaque est ensuite lavée 5 fois avec du PBS-Tween.

* dépôt du substrat de la peroxydase :

On dépose 100 µl d'ABTS préparé comme précisé ci-dessus (C) ; au bout de 5 minutes, les puits correspondant à une forte concentration de oLH, sont colorés en vert.

Après 15 minutes d'incubation à 37°C, une première mesure de la densité optique est effectuée pour fournir une première indication de développement de la réaction. Après quoi, la plaque est à nouveau mise à incuber 15 minutes à 37°C, si la réaction nécessite d'être poursuivie, notamment dans le cas d'échantillons à faible teneur en LH. Puis, on procède à une lecture définitive des résultats. On peut arrêter la réaction enzymatique en ajoutant 20 µl de SDS à 10 % par puits.

La lecture de la densité optique se fait à l'aide d'un spectrophotomètre automatique pour plaques ELISA, muni d'un filtre à 405 nm.

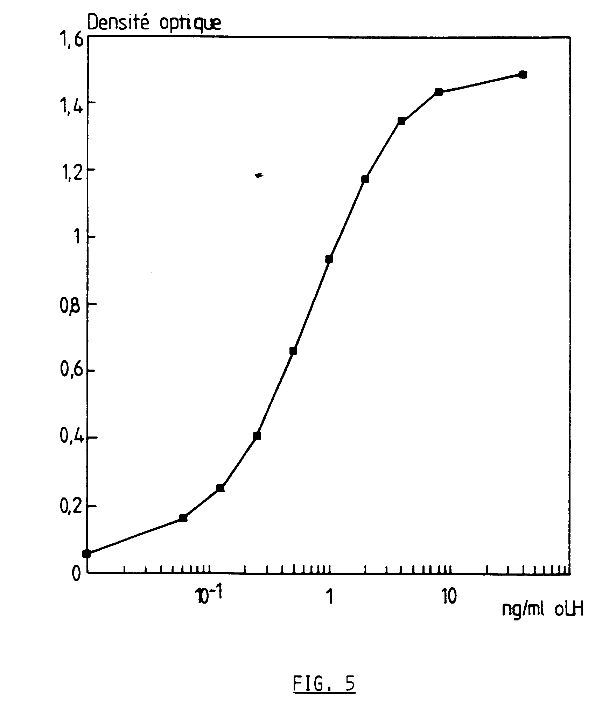

La figure 4 présente les résultats d'un tel dosage réalisé en présence de milieu INC, avec une gamme de concentrations de oLH faite dans du PBS-BSA-Tween (--■--) et avec une gamme réalisée dans du LH (sérum d'animal hypophysectomisé) dilué au 1/10e avec du PBS-BSA-Tween (-■-). La superposition parfaite des deux courbes indique que l'interférence non-spécifique décrite ci-dessus est totalement éliminée. On constate également une amélioration de la sensibilité du dosage par rapport à la courbe (-■-) de la figure 3.

L'effet du milieu INC est le même, qu'il soit composé de 50 % de plasma ou de sérum d'animal hypophysectomisé.

La proportion de 50 % de LH⁻ dans le milieu INC n'est pas impérative et peut être augmentée. En dessous de 50 % cependant, l'interférence non-spécifique décrite n'est plus totalement éliminée.

La figure 5 illustre la courbe obtenue, dans ces conditions, avec une gamme standard préparée dans le milieu de dilution indiqué ci-dessus. (La figure 5 a été obtenue dans les mêmes conditions expérimentales que la courbe (--■--) de la figure 4. Le seuil de détection du dosage est de 60 pg/ml. Le coefficient de variation intra-dosage est de 2,5 % (n = 10), le coefficient de variation inter-dosage est de 6 % (n = 8).

Le protocole exposé ci-dessus est un mode de réalisation non limitatif tant pour les concentrations d'anticorps que pour les temps d'incubation du substrat que l'on peut varier. Les conditions expérimentales présentées ci-dessus représentent un mode de réalisation préféré pour le développement d'un dosage très sensible et très fiable (peu de variabilité intra et inter-dosage) tout en utilisant les plus faibles concentrations en réactifs possibles.

1.b) Révélation de la peroxydase avec l'OPD :

L'OPD peut être utilisé comme substrat, avec le même protocole expérimental. On distribue 100 µl de substrat par puits ; la révélation enzymatique se fait à température ambiante ou à 4°C pendant 5 à 20 mn ; elle est arrêtée avec 50 µl de solution acide comme précisé ci-dessus (C). La lecture de la densité optique se fait à 492 nm, à l'aide du même type d'appareil.

Ce dosage est également utilisable dans les milieux de culture de cellules et le lait notamment.

14

**EXEMPLE 4 : Dosage fluorimétrique quantitatif avec le système AP2-β-galactosidase : dosage de LHs animales**

*   Sensibilisation des plaques (coating) :

    L'anticorps anti-LH lapin (AP1) est préparé à la concentration de 2,5 µg/ml dans du tampon carbonate 0,1 M, pH 9,6. Il est distribué à raison de 100 µl par puits. L'incubation est de 1 heure à 37°C puis 18 heures à 4°C.

*   surcoating ou saturation des puits :

    Après 5 lavages au PBS-Tween, 100 µl de milieu INC sont déposés dans chaque puits. L'incubation est de 30 mn à 37°C. La plaque est, soit conservée en milieu sec, soit directement utilisée.

*   Incubation des échantillons :

    Elle se fait de la même façon que dans le protocole exposé à l'Exemple 3. La durée d'incubation peut varier de 1 h à 1 h 30.

*   Incubation du deuxième anticorps (anti-LH de cheval couplé à la β-galactosidase, AP2) :

    Après lavages de la plaque, l'AP2-β-galactosidase est déposé à raison de 100 µl par puits. Il a été préalablement préparé dans le milieu INC à la dilution de 1 µg/ml et préincubé 30 mn à 37°C.

    L'incubation dans les puits est de 1 h à 37°C après quoi la plaque est lavée 5 fois avec du PBS-Tween.

*   Révélation de la β-galactosidase avec un substrat fluorogénique (4-MUG) :

    200 µl de MUG préparé selon les indications données ci-dessus en C sont déposés dans chaque puits. La plaque est incubée à 42°C pendant 1 à 2 heures. La mesure de la fluorescence se fait avec un fluorimètre automatique pour plaques ELISA ("Microfluor" de DYNATECH - USA).

    Sa longueur d'onde d'excitation est de 360 nm ; il enregistre à une longueur d'onde de 448 nm.

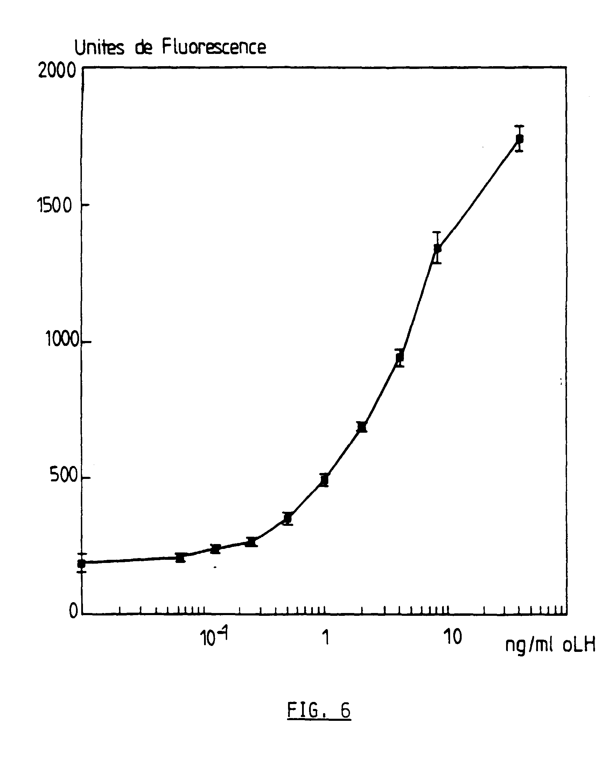

    Les résultats obtenus avec une gamme standard réalisée dans le milieu ABS-BSA-Tween-LH$^-$ 1/10e sont rapportés sur la figure 6. Le MUG a été incubé pendant 1 heure ; on observe que le seuil de détection est moins bon que dans le protocole de l'Exemple 3 (entre 250 et 500 pg/ml). D'autre part, les coefficients de variation intra et inter-dosage sont plus élevés dans ce cas. L'intérêt de ce protocole est de permettre de réaliser le dosage en un temps plus court.

    Les concentrations de réactifs ont été choisies de sorte que le rapport quantité de réactifs utilisés/qualité du dosage soit le plus favorable. Il est évident que les concentrations peuvent être modifiées tout en gardant un dosage correct.

**EXEMPLE 5 : Dosage de LHs animales avec le système AP2-peroxydase**.

On peut réaliser dans les mêmes conditions que celles de l'Exemple 4, un dosage de LH, dans lequel AP2 est couplé à une peroxydase.

**EXEMPLE 6 : Dosage colorimétrique quantitatif et qualitatif de la LH, réalisé avec le système AP3-peroxydase à partir du rang ; développement d'un kit de détection du pic de LH chez les animaux, utilisé en laboratoire ou sur le terrain :**

Le dosage à partir du sang peut être utilisé dans un but tant quantitatif que qualitatif ; on entend par "dosage qualitatif", un test permettant la détection du pic préovulatoire de LH chez la femelle, sur le terrain, sans appareil de mesure, simplement interprétable à l'oeil. Ce test permet de même la détection de pulses de LH chez la femelle ou chez le mâle.

a) Procédé de collecte du sang :

Un dosage réalisé sur du plasma présente une véritable charge dans la mesure où il nécessite une étape de centrifugation, le pipetage du plasma après centrifugation et son transfert dans de nouveaux tubes et une étape de dilution avant l'utilisation dans le dosage (c'est-à-dire pipetage, homogénéisation dans le tampon de dilution).

Afin d'éliminer ce problème, on applique dans ce test, un procédé nouveau de prélèvement de sang sur les animaux. Il consiste à placer une aiguille très fine dans la veine jugulaire de l'animal, laisser perler une goutte de sang, y appliquer l'extrémité d'un capillaire en verre, hépariné, et calibré à un volume bien précis (10 ou 25 µl). Le capillaire doit être tenu horizontalement, afin que le sang puisse migrer jusqu'à l'autre extrémité du capillaire. A l'aide d'une petite poire en plastique, adaptée au diamètre du capillaire, le contenu de celui-ci est récupéré dans un tube contenant 225 µl de PBS-Tween-BSA hépariné dans le cas d'un capillaire de 25 µl. Le sang est ainsi dilué 10 fois et n'a plus qu'à être distribué en 2 fois 100 µl dans les puits prévus d'une plaque.

On peut éviter cette dernière étape, en remplissant tous les puits de la plaque avec 90 µl de PBS-BSA-Tween hépariné. Il suffit alors d'utiliser des capillaires de 10 µl de volume et de les vider chacun directement dans un puits de la plaque de microtitration. L'échantillon à doser est ainsi directement dilué et déposé dans la plaque.

b) Protocole :

\* Sensibilisation des plaques :

L'AP1 est dilué à 7,5 µg/ml dans du tampon carbonate 0,1 M pH 9,6 et incubé 1 h à 37°C puis 18 h à 4°C. Les plaques sont ensuite lavées au PBS-Tween, 5 fois, puis remplies de 100 µl par puits de milieu INC. Elles sont incubées 30 mn à 37°C, vidées et séchées puis scellées sous plastique, ou directement utilisées.

\* Dépôt des échantillons :

- soit les 10 µl de sang sont déposés directement dans le puits correspondant à l'échantillon après avoir préalablement rempli tous les puits de 90 µl de PBS-BSA-Tween hépariné ;
- soit on dépose 100 µl par puits des 25 µl de sang dilués dans 225 µl de PBS-BSA-Tween hépariné.

L'incubation est de 1 heure ou moins à 37°C ou à la température ambiante. Pendant l'incubation, on agite la plaque toutes les 15 mn, en la basculant manuellement afin de réhomogénéiser les hématies dans la phase liquide. On effectue ensuite 5 lavages au PBS-Tween si on est en laboratoire, ou au sérum physiologique (NaCl 0,15 M) ou à l'eau courante du robinet si on est sur le terrain.

\* Dépôt du deuxième anticorps :

100 µl d'AP2 préparé dans le milieu INC à 5 µg/ml sont déposés dans chaque puits. L'incubation est de 45 mn à 37°C ou à la température ambiante. La plaque est ensuite lavée selon les indications précédentes.

\* Dépôt du troisième anticorps :

100 µl d'AP3-peroxydase sont déposés par puits ; l'AP3 a été préparé dans le milieu INC à la dilution 1/5000e selon les indications de l'Exemple 3.

Dans le kit notamment, AP2 et AP3 seront fournis tout préparés. Après une incubation de 45 mn à 37°C ou à température ambiante, la plaque est lavée comme précédemment et on procède à la révélation enzymatique.

c) Lecture du dosage à l'oeil nu, interprétation des observations :

La solution d'ABTS (préparée selon les indications ci-dessus en C) et l'eau oxygénée à 2 % ne sont mélangées qu'extemporanément c'est-à-dire juste avant utilisation. 100 µl de substrat sont déposés par puits.

La réaction enzymatique est instantanée et se fait à température ambiante. Au bout de 2 mn, l'apparition d'une couleur verte dans les puits correspondant à un pic préovulatoire de LH est visible. Au bout de 5 mn, on distingue les prélèvements de sang "porteurs" d'un pic préovulatoire de LH comparativement aux autres dont le substrat reste incolore.

\* Ce test de type "tout ou rien" est possible grâce à la combinaison AP1-AP2 et milieu INC, qui neutralise tout signal non-spécifique, c'est-à-dire que les échantillons à faible teneur en LH, préparés dans ce protocole, n'entraînent aucune coloration du substrat contrairement à ceux de forte teneur en LH qui provoquent l'apparition instantanée d'une couleur verte.

Le milieu INC évite ainsi d'avoir à distinguer un vert plus ou moins clair (cas d'un signal non spécifique élevé, par exemple) d'un vert foncé (niveau de LH élevé) avec tous les risques de variabilité que cela représente. En effet, le caractère de tout ou rien de ce test présente un avantage important par rapport aux kits immunoenzymatiques vendus pour la LH humaine ou pour d'autres hormones animales.

d) Dosage quantitatif de LH à partir du sang :

Le dosage réalisé à partir du sang peut être à la fois qualitatif mais aussi quantitatif. Il suffit d'appliquer le protocole expérimental optimisé du dosage, exposé ci-dessus à l'Exemple 3 et de prendre la précaution d' "agiter" la plaque pendant l'incubation du sang.

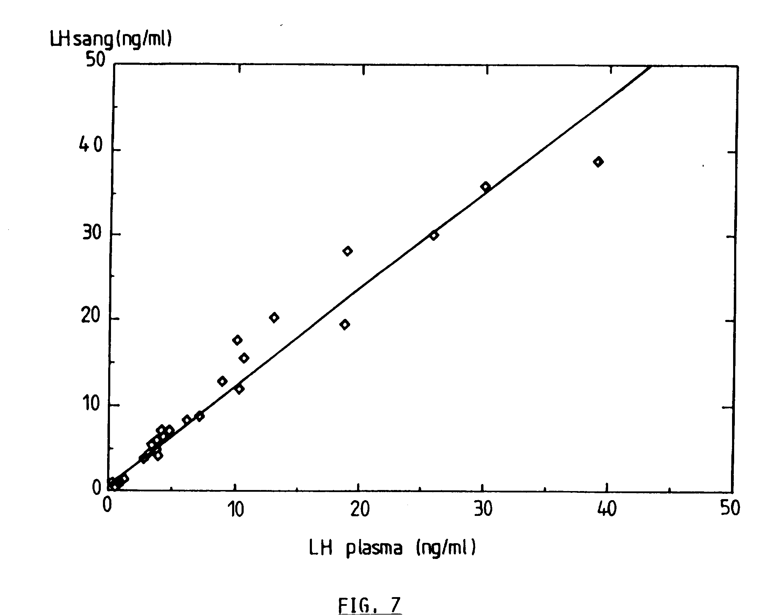

Le calcul du coefficient de corrélation entre la mesure de la concentration de LH dans le sang et dans le plasma provenant du même prélèvement, a été réalisé en comparant les résultats obtenus à partir des deux "milieux biologiques" (voir figure 7).

Le coefficient de corrélation = 0,9788 (n=40), l'équation de la droite de régression : y = 1,14 x + 0,889 où Y est la concentration de LH (ng/ml) mesurée dans le sang et X est la concentration de LH (ng/ml) mesurée dans le plasma selon le protocole exposé à l'Exemple 3.

La valeur obtenue pour le coefficient de corrélation indique qu'un dosage quantitatif très précis à partir du sang est tout à fait possible avec le protocole indiqué ci-dessus.

**EXEMPLE 7 : Autres variantes du test de détection de LHs animales conforme à l'invention** :

L'incubation simultanée d'AP2 et d'AP3-peroxydase (1 h) diminue la durée totale du dosage.
Les essais réalisés ont donné une courbe dose-réponse de moins bonne sensibilité que celle obtenue selon le protocole exposé ci-dessus mais suffisante pour la détection de fortes concentrations en LH (à partir de 15 ng/ml). L'incubation du substrat doit en contre-partie être plus longue 30 à 45 mn si nécessaire pour la détection de pics préovulatoires.

**EXEMPLE 8 : Détection du pic de LH dans le lait chez la chèvre et la brebis :**

1) chez la chèvre:

L'exemple illustré ici a été réalisé sur un troupeau de 20 chèvres synchronisées pour traitement d'insémination artificielle.

Le protocole expérimental est le même que celui exposé dans l'exemple 3, à la différence que 100 μl de lait sont déposés dans chaque puits. Il n'y a dans ce cas aucune dilution à faire, le lait est utilisé brut, non dilué, contrairement au plasma ou au sang.

Après avoir éliminé les premiers jets du lait, les 100 μl d'échantillon peuvent être prélevés avec un capillaire calibré, de la même façon que dans le cas du sang. De même, la plaque est lavée au PBS-Tween ou à l'eau du robinet selon l'endroit de la réalisation du dosage.

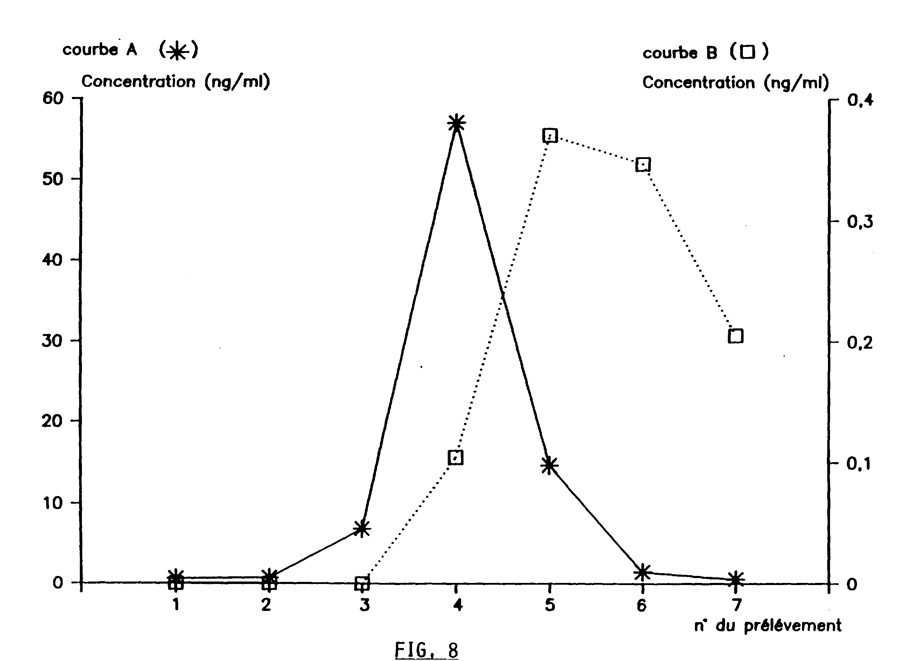

La figure 8 présente le profil de sécrétion du pic de LH chez une chèvre, dans le sang (courbe -*-) et dans le lait (..□..). On constate que les concentrations de LH trouvées dans le lait sont infimes par rapport aux concentrations plasmatiques. Pour cette raison, il est nécessaire d'utiliser un spectrophotomètre de plaques de microtitration. On constate que l'apparition du pic de LH dans le lait est décalée de 4 heures par rapport au sang, si on utilise le début du pic comme point de comparaison. Le Tableau I ci-dessous rapporte les concentrations de LH mesurées dans le sang et le lait au cours de 7 prélèvements effectués toutes les 4 heures dans les deux milieux simultanément. La comparaison des résultats montre que l'apparition du pic de LH dans le lait est décalée de 4 ou 8 heures par rapport au sang, si on compare le maximum du pic. Le début du pic, par contre, est toujours décalé de 4 heures dans le lait par rapport au sang, et ce de façon constante. En dehors du pic préovulatoire, les concentrations de LH dans le lait ne sont pas détectables.

Détection du pic de LH chez la Chèvre dans le sang et dans le lait

| Chèvre n° | Prélèvements | | | | | | | | | | | | | |
|---|---|---|---|---|---|---|---|---|---|---|---|---|---|
| | n° 1 | | n° 2 | | n° 3 | | n° 4 | | n° 5 | | n° 6 | | n° 7 | |
| | Sang | Lait | Sang | Lait | Sang | Lait | Sang | Lait | Sang | Lait | Sang | Lait | Sang | Lait |
| 1 | 0.10 | ND | 0.30 | ND | 0.34 | ND | 33.50 | ND | 24.00 | 0.058 | 1.20 | 0.125 | 0.60 | 0.093 |
| 3 | 0.10 | ND | 0.20 | ND | 0.15 | ND | 0.28 | ND | 46.14 | 0.050 | 8.85 | 0.136 | 0.86 | 0.160 |
| 5 | 0.92 | ND | 0.50 | ND | 1.03 | ND | 53.90 | ND | 35.45 | 0.231 | 2.37 | 0.319 | 0.83 | 0.148 |
| 6 | 0.30 | ND | 0.29 | ND | 1.02 | ND | 1.57 | ND | 54.30 | 0.080 | 5.88 | 0.157 | 0.86 | 0.119 |
| 7 | 0.60 | ND | 0.38 | ND | 6.91 | ND | 57.00 | 0.104 | 14.66 | 0.370 | 1.50 | 0.346 | 0.54 | 0.205 |
| 8 | 0.30 | ND | 0.32 | ND | 0.52 | ND | 1.07 | ND | 15.13 | ND | 24.18 | 0.043 | 1.75 | 0.120 |
| 10 | 0.21 | ND | 0.23 | ND | 0.37 | ND | 0.98 | ND | 44.68 | ND | 41.73 | 0.142 | 2.12 | 0.197 |
| 11 | 0.10 | ND | 0.10 | ND | 0.23 | ND | 60.00 | 0.050 | 11.43 | 0.163 | 1.20 | 0.200 | 0.65 | 0.133 |
| 14 | 0.80 | ND | 0.72 | ND | 0.65 | ND | 3.28 | 0.050 | 29.55 | 0.060 | 43.08 | 0.090 | 2.21 | 0.210 |
| 15 | 0.23 | ND | 0.36 | ND | 0.20 | ND | 1.71 | ND | 17.30 | ND | 39.63 | 0.117 | 2.19 | 0.257 |
| 16 | 1.51 | ND | 1.32 | ND | 1.78 | ND | 1.10 | ND | 1.03 | ND | 0.80 | ND | 0.48 | ND |
| 17 | 0.30 | ND | 0.54 | ND | 0.60 | ND | 3.47 | ND | 45.42 | 0.056 | 6.17 | 0.271 | 0.20 | 0.185 |
| 18 | 0.30 | ND | 0.36 | ND | 0.68 | ND | 49.59 | ND | 19.96 | 0.114 | 2.75 | 0.197 | 0.30 | 0.097 |
| 20 | 0.16 | ND | 0.27 | ND | 57.90 | ND | 17.39 | 0.099 | 2.92 | 0.106 | 0.57 | 0.009 | 0.11 | 0.050 |

ND: non détectable
Les concentrations sont données en ng/ml

TABLEAU I

EP 0 533 719 B1

Ce Tableau I illustre bien l'importance de la mise en oeuvre du procédé conforme à l'invention dans la conduite de la reproduction à l'intérieur des troupeaux caprins mais également et ce de manière plus générale pour toutes les espèces pour lesquelles il est utilisable (bovins, ovins, etc...). Il permet d'éliminer les femelles n'ayant pas eu de pic préovulatoire (donc n'ayant pas ovulé) et de féconder à un moment optimum les femelles ayant présenté un pic préovulatoire précoce. Ceci permet l'établissement de schémas d'insémination tels que le schéma III ci-dessous, très utile notamment pour rentabiliser au mieux les campagnes d'insémination artificielle menées dans les différentes espèces animales concernées.

**°VARIABILITE DE L'INTERVALLE FIN DE TRAITEMENT ⟷ PIC DE LH°**

| Intervalle fin de traitement pic de LH (h) | 16h | 20h | 24h | 28h | 32h | 36h | 40h | I.A. 45H non détecté à 40h |
|---|---|---|---|---|---|---|---|---|
| nombre de femelles | (1) | (2) 17,5% | (7) | (9) | (12) 63,2% | (9) | (6) | (11) 19,3% |
| taux de gestation | (0/1) | (2/2) 40% | (2/7) | (9/9) | (8/12) 80,5% | (7/9) | (6/6) | (5/11) 45,5% |

## SCHEMA III

<u>2) Chez la brebis</u> :

L'exemple illustré ici a été réalisé sur un troupeau de 12 brebis laitières Lacaune choisies parmi les "bonnes" laitières.

Ces femelles ont reçu un traitement de synchronisation (acétate de fluorogestone), pendant 14 jours, après quoi elles ont reçu une injection de 500 U.I. d'eCG (hormone stimulant l'ovaire).

Les prélèvements de sang et de lait ont été faits toutes les quatre heures, à partir de la détection du comportement d'oestrus. Chaque femelle a été ainsi prélevée neuf fois, pendant 32 heures.

La mesure de la concentration de LH s'est faite dans les deux milieux, sang et lait, selon le protocole expérimental exposé dans l'exemple 3 ci-dessus.

Le tableau II ci-après exprime les résultats en ng/ml, et montre un parfait décalage de quatre heures entre le pic de LH dans le sang et celui détecté dans le lait, dans lequel, en dehors du pic préovulatoire, les concentrations en LH ne sont pas détectables.

**Détection du pic de LH chez la brebis dans le sang et dans le lait (TABLEAU II)**

Prélèvements

| Brebis n° | n°1 Sang | n°1 Lait | n°2 Sang | n°2 Lait | n°3 Sang | n°3 Lait | n°4 Sang | n°4 Lait | n°5 Sang | n°5 Lait | n°6 Sang | n°6 Lait | n°7 Sang | n°7 Lait | n°8 Sang | n°8 Lait | n°9 Sang | n°9 Lait |
|---|---|---|---|---|---|---|---|---|---|---|---|---|---|---|---|---|---|---|
| 1 | 1,63 | - | 1,64 | - | 1,59 | - | 27,11 | - | 34,37 | 0,62 | 4,32 | 0,28 | 1,22 | 0,55 | 0,97 | 0,23 | 0,73 | 0,25 |
| 2 | 0,93 | - | 2,27 | - | 35,89 | 0,07 | 39,65 | 0,6 | 6,9 | 0,97 | 1,41 | 0,7 | 1,05 | 0,31 | 2,61 | 0,89 | 0,31 | 0,87 |
| 3 | 0,7 | - | 1,5 | - | 25,67 | - | 15,23 | 0,24 | 1,74 | 0,2 | 0,9 | 0,2 | 0,3 | 0,88 | 0,55 | - | 0,56 | - |
| 4 | 0,49 | - | 0,34 | - | 36,22 | - | 7,15 | 0,19 | 1,39 | 0,17 | 0,55 | 0,15 | 0,2 | 0,83 | 0,2 | - | 0,2 | - |
| 5 | 0,37 | - | 0,16 | - | 8,36 | - | 0,99 | - | 37,88 | - | 14,4 | 0,4 | 2,66 | 0,4 | 1,38 | 0,31 | 0,23 | 0,21 |
| 6 | 0,58 | - | 0,52 | - | 0,66 | - | 1,69 | - | 18,28 | - | 9,3 | 0,23 | 1,07 | 0,17 | 0,85 | 0,13 | 0,3 | 0,88 |
| 7 | 0,15 | - | 0,23 | - | 19,5 | - | 0,5 | 0,25 | 1,2 | 0,26 | 0,3 | 0,12 | 0,2 | - | 0,15 | - | 0,25 | - |
| 8 | 0,27 | - | 0,33 | - | 0,3 | - | 0,6 | - | 23 | - | 15 | 6,52 | 2,15 | 0,41 | 0,2 | 0,3 | 0,12 | 0,13 |
| 9 | 0,38 | - | 36,8 | - | 14,20 | 0,32 | 1,72 | 0,22 | 0,68 | 0,18 | 6,25 | 0,39 | 0,31 | 0,85 | 2,57 | - | 0,31 | - |
| 10 | 2,4 | - | 3,76 | - | 37,96 | 0,88 | 38,31 | 0,41 | 6,88 | 0,38 | 3,26 | 3,19 | 2,45 | 0,13 | 0,37 | 0,88 | 2,1 | - |
| 11 | 0,37 | - | 0,55 | - | 26,86 | 0,86 | 4,23 | 0,12 | 5,14 | 0,12 | 1,64 | 0,89 | 0,47 | 0,86 | 0,49 | 0,87 | 0,48 | - |
| 12 | 2,31 | - | 37,1 | 0,15 | 16,66 | 0,21 | 6,36 | 0,17 | 1,53 | 0,11 | 0,46 | 0,89 | 0,72 | 0,88 | 0,74 | - | 0,67 | - |

Production laitière/jour : 0,98 ; 1,62 ; 1,82 ; 1,94 ; 1,3 ; 1,94 ; 1,76 ; 1,26 ; 1,68 ; 1,54 ; 0,98 ; 1,18

valeurs soulignées : valeur maximale du pic de LH dans le sang et dans le lait

LH : valeurs en ng/ml

La détection du pic de LH dans le lait offre plusieurs avantages :

. sa durée est plus importante que dans le sang (20 à 24 heures contre 8 à 12 heures dans le sang), ce qui nécessite moins de prélèvements à faire, l'intervalle entre chaque prélèvement pouvant être plus important (par exemple toutes les huit heures) ;

. une prise de lait est moins "stressante" qu'une prise de sang, et peut se faire au moment de la traite des animaux ;

. de plus, chez la brebis, la lecture du test dans le lait peut se fait à l'oeil nu.

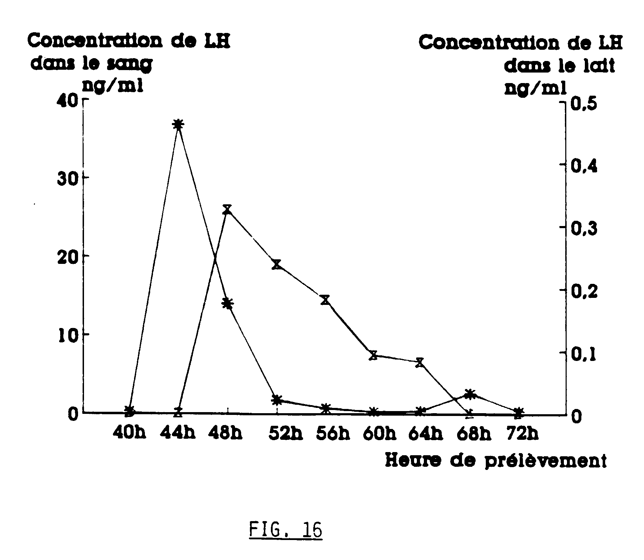

La figure 16 représente le profil de sécrétion du pic de LH en ng/ml, en fonction du temps, chez une brebis, dans le sang, (courbe -*-) et dans le lait ($-\Sigma-$ ).

**EXEMPE 9 : Applications sur le terrain :**

Ces exemples sont illustrés par des planches et appuyés par une étude de la corrélation entre les concentrations obtenues par le dosage ELISA et celles obtenues par un dosage radioimmunologique réalisé dans le plasma.

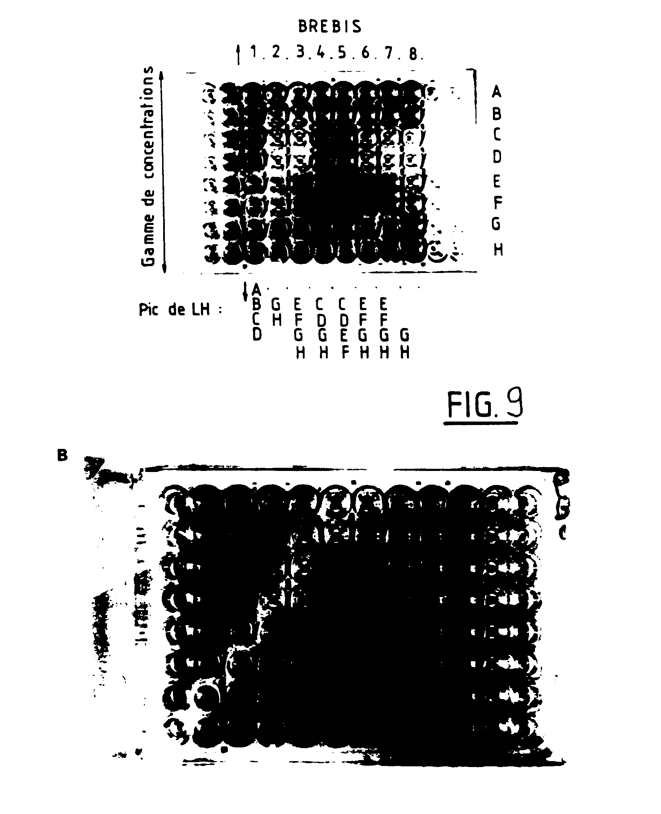

a) Détection de pics préovulatoires de LH chez des brebis superovulées (figures 9A et B) :

Les figures 9 sont des photos de plaques comportant 12 colonnes dénommées 1 à 12 et 8 lignes dénommées A à H.

Les colonnes 1 et 2 correspondent à la gamme étalon des concentrations et les colonnes 3 à 11 correspondent aux prélèvements réalisés chez huit brebis.

La figure 9A donne l'ordre de dépôt des échantillons. Les prélèvements d'une femelle sont déposés en colonne (colonnes 3 à 11) ; chaque prélèvement est dosé en double. Les pics de LH "apparaissent" en vert foncé ce qui dans cette figure et les figures suivantes est représenté en gris foncé comme on le voit en détail sur la figure 9B, dans laquelle la gamme de concentrations est également déposée à gauche de la plaque sur 2 colonnes (colonnes 1 et 2).

Les prélèvements ont été réalisés toutes les 4 heures à partir du début des chaleurs. Pour la brebis 1 (colonne 3), on observe un pic sur les deux premiers prélèvements (puits $A_3$-$B_3$ et $C_3$-$D_3$).

Ce dosage a été réalisé à partir de plasmas. Le temps de révélation a été de 15 mn à température ambiante.

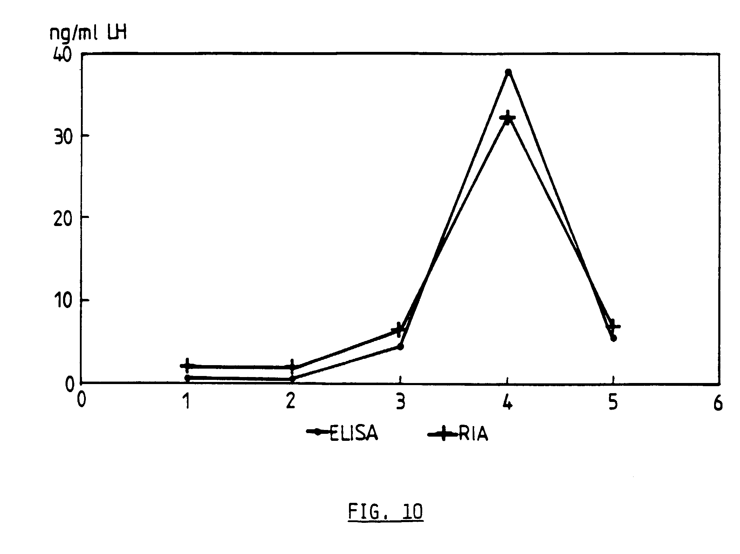

La figure 10 illustre un profil de sécrétion de LH obtenu chez une brebis (Exemple 3) par le dosage ELISA (AP3-peroxydase/ABTS) et par RIA (radio immuno-assay) à partir du plasma.

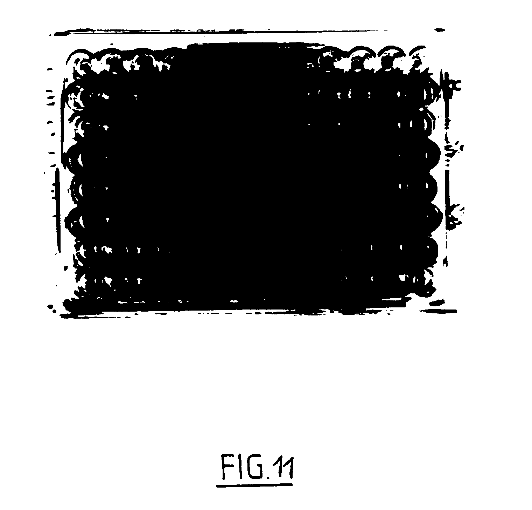

b) Détection de pics préovulatoires chez des chèvres superovulées (figure 11) :

Cette figure représente une photo d'une plaque du même type que la précédente (colonnes 1 à 12, ligne A à H).

Des prélèvements ont été réalisés toutes les 4 heures après le début des chaleurs.

Les dosages ont été faits à partir du sang. La révélation a été de 10 mn à température ambiante.

Les prélèvements par animaux ont été déposés en colonne : la colonne 2 montre, par exemple, un pic dans les puits $D_2$-$E_2$ puis la fin du pic en $F_2$. Le sommet du pic est en $D_2$. En dehors des pics, le substrat reste incolore. Dans la colonne 6, on observe de même le maximum d'un pic en $D_6$-$E_6$ puis la fin du pic en $F_6$.

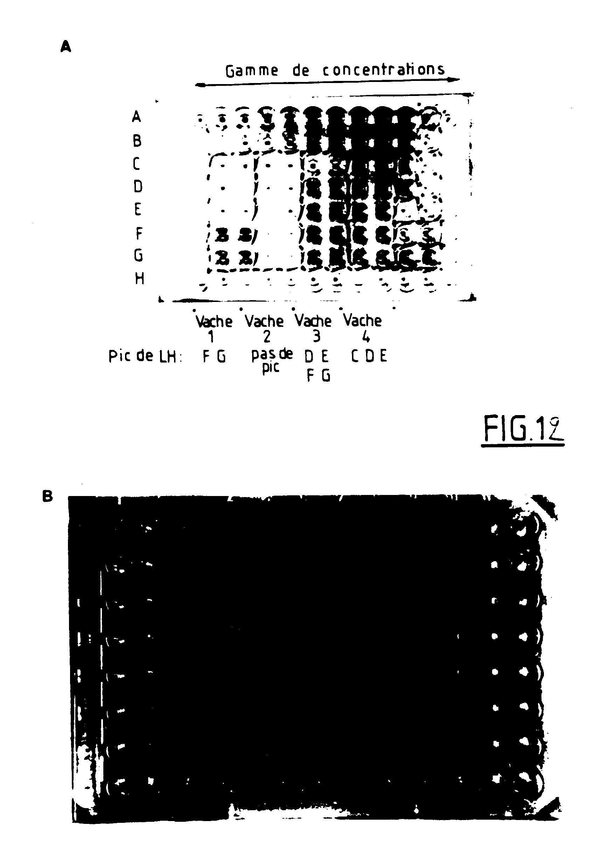

c) Détection de pics préovulatoires chez des vaches (figure 12) :

Après injection de GnRH, on prélève les animaux toutes les 30 minutes. Les prélèvements sont dosés en double et déposés en colonne (vache 1 ; colonnes 2 et 3 ; vache 2 : colonnes 4 et 5 ; vache 3 : colonnes 6 et 7 ; vache 4 : colonnes 8 et 9. La gamme standard est déposée dans les deux premières lignes A et B (figure 12A).

Le dosage est fait à partir du sang. Le temps de révélation est de 5 mn en figure 12A et de 15 mn en figure 12B à température ambiante. On constate l'absence de pic de LH chez la vache 2, un début de pic de LH au 4e et 5e prélèvement ($F_2$-$F_3$/$G_2$-$G_3$) pour la vache n° 1. Chez les deux animaux suivants, le pic est apparu plus rapidement : au 2e prélèvement ($D_6$-$D_7$) pour la vache n° 3 et dès le 1er prélèvement ($C_8$-$C_9$) pour la vache n° 4. On observe très bien la retombée du pic pour cette dernière.

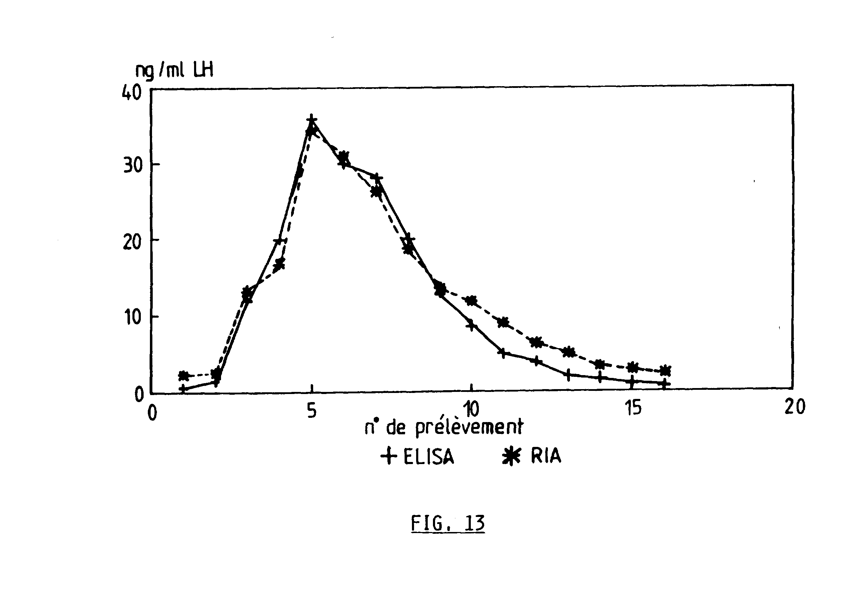

La figure 13 donne l'intégralité du profil de sécrétion de la LH chez une vache traitée au GnRH. On peut constater une superposition des concentrations mesurées par ELISA et par RIA.

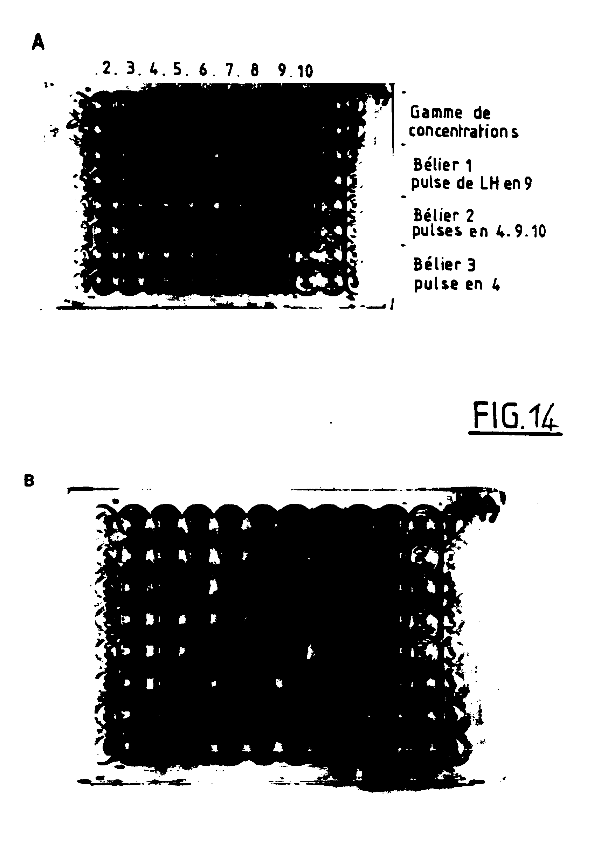

d) Etude de la micropulsatilité chez des béliers (figure 14) :

Les dosages ont été réalisés dans le plasma selon le protocole de l'Exemple 3. Les prélèvements ont eu lieu toutes les 20 minutes. Ils sont déposés par ligne (bélier 1 : lignes C et D ; lignes E et F ; bélier 3 : lignes G et H) et dosés en duplicates (les duplicates sont disposés verticalement).

Le temps de révélation est de 30 minutes. On observe un pulse en $C_9$-$D_9$ pour le bélier n° 1, en $E_4$-$F_4$ et $E_{10}$-$F_{10}$ pour le bélier n° 2, et un très léger en $G_4$-$H_4$ pour le bélier n° 3 de même en $E_9$-$F_9$ pour l'animal n° 2.

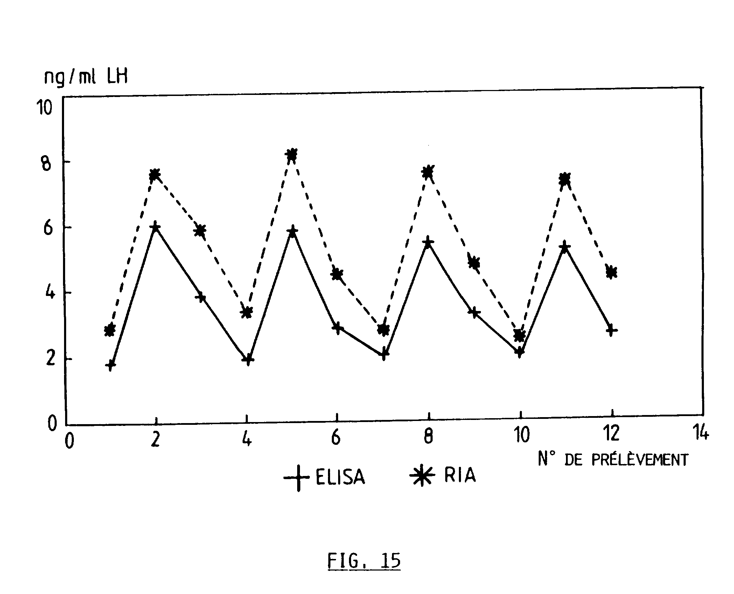

La figure 15 illustre un profil de sécrétion de LH obtenu chez le bélier avec les dosages ELISA et RIA. Les concentrations obtenues en ELISA ont des valeurs absolues plus basses que celles en RIA, par contre, le profil est identique. Cette différence est due à la meilleure sensibilité du dosage ELISA qui permet de détecter des valeurs plus faibles que le RIA.

e) Calcul du coefficient de corrélation obtenu entre dosages réalisés par ELISA et dosages par RIA :

Le dosage RIA utilisé est celui publié par J. PELLETIER et al., 1982.

1) Corrélation chez les bovins : (n = 60)

. coefficient de corrélation : r : 0,9705
. équation de la droite de régression :

$$y = 1{,}09x - 0{,}971.$$

2) Corrélation chez les caprins : (n = 60)

. coefficient de corrélation : r = 0,9759
. équation de la droite de régression :

$$y = 1{,}031x - 0{,}978.$$

3) Corrélation chez les ovins (n = 67)

. coefficient de corrélation : r = 0,9808
. équation de la droite de régression :

$$y = 1{,}0745x - 2{,}45$$

où Y est la concentration de LH (ng/ml) mesurée par ELISA,
X est la concentration de LH (ng/ml) mesurée par RIA.

**EXEMPLE 10 : Détection du pic de LH chez la biche.**

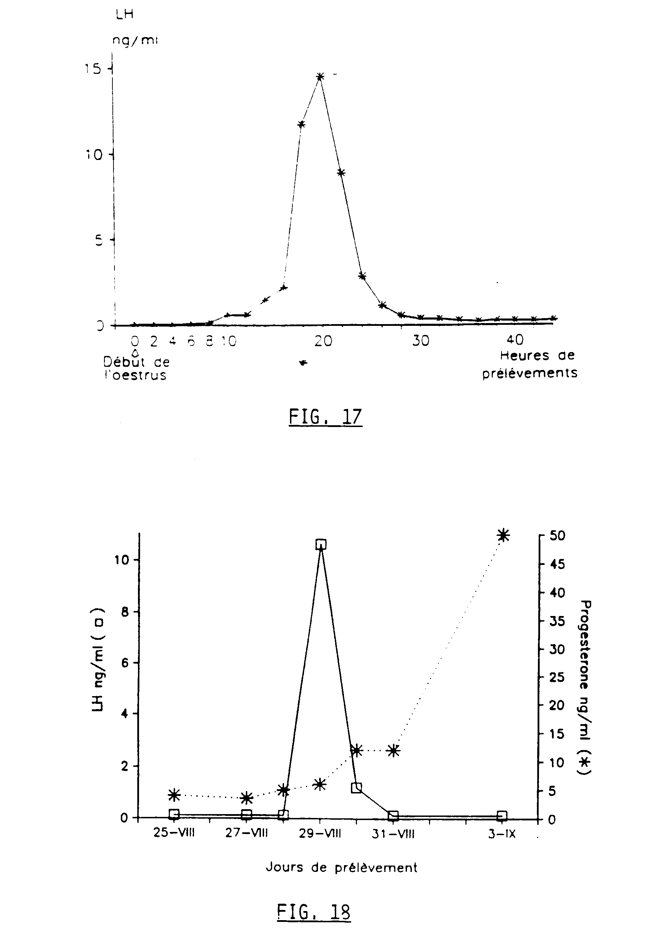

Des dosages ont été fait à partir de prélèvements de sang réalisés toutes les deux heures pendant toute la période des chaleurs, chez la biche Rusa de Nouvelle-Calédonie. Les prélèvements ont été faits sur sept biches et les dosages ont été réalisés comme décrit à l'exemple 3. Les tests sont lus à l'oeil nu comme pour les autres espèces. La figure 17 montre le profil de sécrétion de LH en ng/ml en fonction du temps chez une biche (biche n° 80). Les résultats obtenus chez l'ensemble des femelles montrent là encore, que le moment du pic de LH est très variable pendant la période des chaleurs : il a lieu, en général, au début des chaleurs.

Ceci illustre bien l'importance du procédé conforme à l'invention pour pouvoir établir la précision du moment d'insémination, étant donné la variabilité dans l'apparition du pic de LH.

On peut, en particulier, noter l'intérêt de ce procédé dans l'insémination artificielle, de femelles donneuses d'embryons, chez des races de la famille des cervidés en voie de disparition.

**EXEMPLE 11 : Détection du pic de LH chez la chienne**

Les dosages sont réalisés comme décrit à l'exemple 3, à partir des prélèvements de sang journaliers faits sur 2 chiennes pendant la période des chaleurs. Chez la chienne, le pic préovulatoire de LH s'étend sur un laps de temps plus important que chez les autres espèces décrites : la durée est de 24 heures en moyenne. De ce fait, il peut être détecté en ne faisant qu'un prélèvement de sang journalier, ce qui présente un avantage pour la clientèle d'un cabinet vétérinaire. Les résultats obtenus avec la chienne "DIXIE" illustrés sur la figure 18 le montrent clairement. Les tests sont lus à l'oeil nu comme pour les autres espèces.

La figure 18 représente le profil de secrétion de LH, (-□-) et de progestérone (-*-) en ng/ml, obtenus chez la chienne DIXIE.

On observe qu'au delà de 48 heures après le pic préovulatoire (3.9.90), la teneur en progestérone a considérablement augmenté (>50 ng/ml). Ceci témoigne de la présence d'un corps jaune (responsable de la forte sécrétion en progestérone) qui résulte d'une ovulation.

Etant donné la longueur de la période des chaleurs, chez la chienne, le kit permettra de connaître avec précision le moment de l'ovulation chez la chienne suivie et permettra de pratiquer l'insémination au moment optimal (la fécondation a lieu 48 heures après l'ovulation, chez la chienne). Là encore, un schéma d'insémination pourra être établi précisément, à l'aide du procédé conforme à l'invention.

**EXEMPLE 12 : Détection du pic de LH chez la truie**

Une corrélation a été établie entre les valeurs de concentration en LH établies par un dosage radioimmunologique et celles obtenues avec le procédé conforme à l'invention, réalisé selon le protocole de l'exemple 3. Les dosages ont été faits sur des plasmas de truies.

.   le coefficient de corrélation n = 0,943
.   l'équation de la droite de régression :

$$y = 0,1719x + 0,069 \ (n = 20), \ où :$$

y est la concentration de LH (ng/ml) mesurée par ELISA ; et x est la concentration de LH (ng/ml) mesurée par RIA.

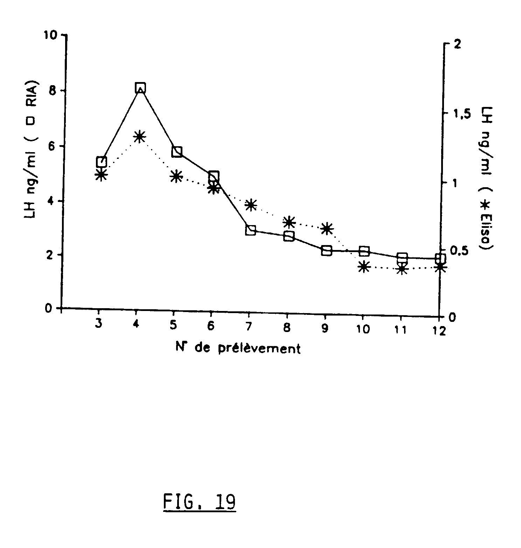

La figure 19 illustre un profil de secrétion de LH en ng/ml obtenu avec un dosage conforme à l'invention (-*-) et avec un dosage RIA (-□-).

**EXEMPLE 13 : Dosage de la FSH ovine avec le procédé conforme à l'invention.**

a- Anticorps utilisés pour le dosage :

-   le premier anticorps ($AC_1$) est un anticorps monoclonal spécifique de la sous-unité $\beta$ de la FSH de nombreuses espèces (bovine, ovine, équine, porcine, humaine, rat, lapin, chien, autruche). Il a été gracieusement fourni par Monsieur le Professeur Jacques LUSSIER pour ces essais expérimentaux. Cet anticorps monoclonal a été produit par le Docteur LUSSIER et son équipe (Université de Montréal - Faculté de médecine vétérinaire - CRRA - CP 5000 - St Hyacinthe J297C6 - Québec). Il s'agit d'un anticorps monoclonal de type IgG1.
-   le deuxième anticorps ($AC_2$) est un anticorps polyclonal spécifique de la FSH ovine, qui a été fait chez le lapin. Il a été produit et est disponible à la station de Physiologie de la reproduction - INRA - 37380 NOUZILLY.
-   le troisième anticorps ($AC_3$) est un anticorps polyclonal commercialisé par les Laboratoires Jackson (USA). Il s'agit d'un anticorps anti-IgG de lapin couplé à la peroxydase et fait chez la chèvre - référence 111-035-003.

b- Protocole expérimental
Le protocole utilisé est celui présenté dans l'exemple 3 :

(1) le premier anticorps est préparé dans du tampon carbonate 0,1M pH 9,6 à la concentration de 7,5 $\mu$g/ml. 100 $\mu$l sont distribués par puits. Les temps d'incubation pour la sensibilisation des plaques sont les mêmes : 1 heure à 37°C et 18 heures à 4°C.
(2) les lavages et la saturation des puits ("surcoating") sont réalisés de la même façon que dans l'exemple 3.
(3) l'incubation des échantillons et de la gamme : même procédé que dans l'exemple 3, la gamme de FSH ovine (standard du NIH : oFSH-13-AFP-2846-C) est préparée dans du PBS-Tween-BSA contenant du sérum d'animal hypophysectomisé dilué au 1/10ème. Elle comprend les concentrations : 125 pg/ml, 250 pg/ml, 500 pg/ml, 1 ng/ml, 2 ng/ml, 4 ng/ml, 8 ng/ml et 40 ng/ml. Elle est déposée en double à raison de 100 $\mu$l par puits. Les plasmas à doser sont dilués dix fois dans du PBS-Tween-BSA. La durée de l'incubation est de une heure à 37°C.
(4) lavages identiques à ceux de l'exemple 3
(5) préparation et incubation du deuxième anticorps ($AC_2$), anti-FSH ovine. Il est préparé dans le milieu INC à la concentration de 5 $\mu$g/ml. Sa préparation est réalisée de la même façon que dans l'exemple 3. L'incubation est de une heure à 37°C.
(6) lavages identiques à ceux de l'exemple 3,
(7) préparation et incubation du troisième anticorps ($AC_3$) anti-IgG de lapin couplé à la peroxydase. Il est préparé selon le protocole de l'exemple 3 à la dilution de 1/5000ème (selon les indications du fabricant). Incubation de une heure à 37°C.
(8) lavages identiques à ceux de l'exemple 3,
(9) dépôt du substrat de la peroxydase et lecture de la densité optique.

c- Résultats :

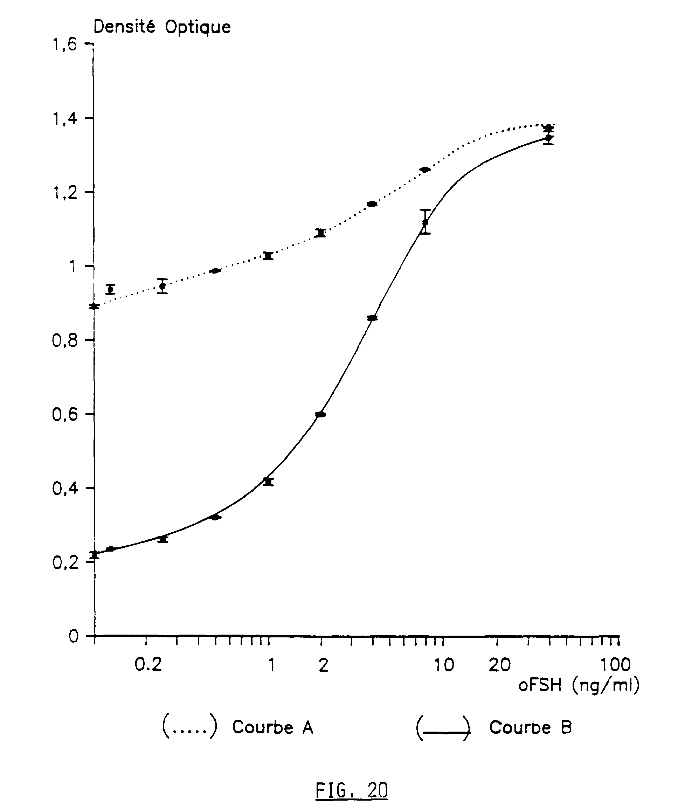

*   la figure 20 représente les résultats d'un tel dosage réalisé selon le protocole INC (courbe B). Le seuil de détection est à 0,25 ng/ml et la sensibilité du dosage est considérablement augmentée comparativement à la courbe A qui- a été obtenue sans appliquer le procédé INC. En effet, dans le cas de la courbe A, le tampon utilisé pour le surcoating, l'incubation de $AC_2$ et $AC_3$ a été du PBS-BSA-Tween simple. Seule la gamme standard de la oFSH a été réalisée dans les mêmes conditions que pour la courbe B, c'est-à-dire dans du PBS-BSA-Tween additionné de sérum de l'animal hypophysectomisé dilué au 1/10éme. La comparaison de ces deux courbes montre l'importance du procédé INC pour l'obtention d'une très bonne sensibilité dans ce dosage de la oFSH. Ces résultats démontrent également que l'efficacité du procédé INC n'est pas restreinte au dosage de LHs animales par les AP1 et AP2 ci-dessus définis mais qu'elle s'applique également au dosage d'une autre hormone hypophysaire, la FSH faisant intervenir d'autres anticorps. L'utilisation du milieu INC a permis, comme dans le dosage de LHs animales et de la hLH, de neutraliser toutes interférences sériques non spécifiques et d'améliorer ainsi considérablement le seuil de détection et la sensibilité du dosage de la oFSH.

* 53 plasmas de brebis ont été dosés pour la FSH selon le procédé INC. Les résultats obtenus par cette technique ont été corrélés à ceux obtenus par un dosage radioimmunologique :

. le coefficient de corrélation est de 0,9759
. l'équation de la droite de régression est

$$Y = 0,5856X - 0,845$$

où Y est la concentration de oFSH (ng/ml) mesurée par ELISA et X est la concentration de oFSH (ng/ml) mesurée par RIA.

Il est à noter enfin, que des concentrations plasmatiques élevées (15 à 20 ng/ml) en oFSH sont tout-à-fait détectables à l'oeil, sans besoin d'appareil de lecture, et se distinguent nettement des taux plasmatiques bas (0,5 à 2 ng/ml). Ceci peut permettre de détecter et mesurer le pic de FSH secrété avant l'ovulation dans des conditions tout-à-fait rustiques, hors du laboratoire.

Par le seuil de détection qu'il permet dans le dosage, le procédé INC donne la possibilité de détecter des niveaux circulants bas (entre 0,25 et 5 ng/ml), ce qui présente un intérêt physiologique évident.

## EXEMPLE 14 : Dosage de la LH humaine

a- Anticorps utilisés pour le dosage :

* le premier anticorps ($AC_1$) est un anticorps monoclonal spécifique de la LH humaine (hLH) commercialisé par la Société Pierce - référence : 37110. Il s'agit d'un anticorps monoclonal de type $IgG_1$, il provient du clone ZMLHZ.
* le deuxième anticorps ($AC_2$) est un anticorps polyclonal anti-hLH, fait chez le lapin et commercialisé par la Société UCB - référence : 1504/001.
* le troisième anticorps ($AC_3$) est un anticorps polyclonal commercialisé par les laboratoires Jackson - référence 111-035-003. C'est un anticorps anti-IgG de lapin couplé à la peroxydase, fait chez la chèvre.

b- Protocole expérimental :
le protocole appliqué est celui de l'exemple 3 :

(1) $AC_1$ est préparé dans du tampon carbonate 0,1 M pH 9,6 à la concentration de 10 μg/ml (ou 7,5 μg/ml), 100 μl sont distribués par puits. L'incubation est de une heure à 37°C et 18 heures à 4°C.
(2) lavages et surcoating (cf exemple 3)
(3) incubation des échantillons et de la gamme : cf exemple 3. Les échantillons à doser sont dilués 10 fois (ou 5 fois) dans du PBS-BSA-Tween. La gamme de hLH est préparée dans du PBS-BSA-Tween additionné de sérum d'animal hypophysectomisé dilué 10 fois. Elle comprend les concentrations suivantes : 62,5 pg/ml, 125 pg/ml, 250 pg/ml, 500 pg/ml, 1 ng/ml, 2 ng/ml, 4 ng/ml, 8 ng/ml, 40 ng/ml.
(4) lavages - cf exemple 3
(5) $AC_2$ est préparé à 2,5 μg/ml et préincubé dans le milieu INC de la même façon que dans l'exemple 3
(6) lavages
(7) $AC_3$ est préparé au 1/5000ème dans le milieu INC, comme dans l'exemple 3
(8) lavages
(9) dépôt du substrat (ABTS) et lecture des résultats au bout d'une heure.

c- Résultats

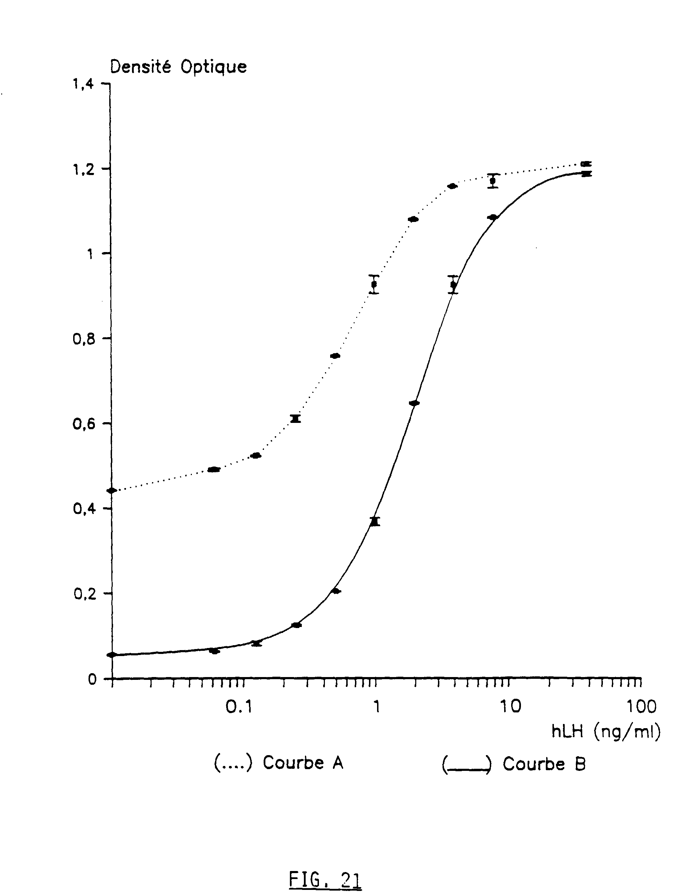

* la figure 21 montre les résultats obtenus avec une gamme standard de hLH en utilisant le procédé INC selon le protocole ci-dessus (courbe B) et ceux obtenus avec une même gamme standard sans appliquer le procédé INC (courbe A). Dans ce dernier cas, le tampon utilisé pour le surcoating, l'incubation de $AC_2$ et $AC_3$ a été du PBS-BSA-Tween seul. La comparaison des deux courbes illustre là encore, l'intérêt et l'impact du procédé INC qui apporte une très nette amélioration dans la sensibiité du dosage et dans son seuil de détection, en neutralisant tout signal non-spécifique dû aux interférences sériques non-spécifiques.

\* 20 plasmas de femmes ont été dosés en hLH selon le procédé ci-dessus présenté. Les résultats obtenus par cette technique ELISA ont été corrélés avec ceux obtenus par le kit de dosage de hLH fabriqué par Abbott (système immuno-enzymo-micro-particulaire - IMX Abbott)

. le coefficient de corrélation est de 0,8808
. l'équation de la droite de régression est

$$Y = 0,0483X +0,0986$$

où Y est la concentration de hLH (ng/ml) mesurée par ELISA et X est la concentration de hLH (UI/l) mesurée par le système IMX Abbott).

\* Etant donné l'absence de tout signal non-spécifique et la grande sensibilité du dosage, il sera possible de discriminer les valeurs hautes (pic préovulatoire de LH) donnant une couleur verte, des valeurs basses restant incolores. Cette interprétation "à l'oeil nu" permettra d'utiliser ce procédé pour la mise au point d'un kit de détection du pic préovulaire de LH chez la femme, à domicile. Il pourra être utilisé aussi pour un dosage quantitatif fiable dans les laboratoires. Ces résultats démontrent que l'efficacité du procédé INC n'est pas restreinte à l'utilisation des anticorps polyclonaux AP1 et AP2 définis ci-dessus dans le doage de LHs animales, mais qu'elle contribue également à l'amélioration du dosage d'une autre LH (la LH humaine) faisant intervenir d'autres anticorps (AG monoclonal anti-hLH, PIERCE ; AC2 polyclonal anti-hLH fait chez le lapin-UCB).

**Revendications**

**Revendications pour les Etats contractants suivants : AT, BE, CH, LI, DE, DK, FR, GB, IT, LU, NL, SE**

1. Procédé de détection et/ou de dosage immunologique d'hormones hypophysaires humaines ou animales, dans des milieux de culture ou dans des fluides biologiques, mettant en oeuvre au moins deux anticorps, spécifiques de l'hormone à doser, lequel procédé est caractérisé en ce que pour augmenter la sensibilité et la spécificité de ladite détection et/ou dudit dosage, lesdits anticorps sont, -préalablement à leur mise en oeuvre dans ladite détection ou ledit dosage-, préincubés dans un milieu contenant du plasma ou du sérum dépourvu de l'hormone à détecter et/ou à doser, obtenu par hypophysectomie (milieu INC).

2. Procédé selon la revendication 1, caractérisé en ce que ledit milieu INC est associé à un tampon à pH neutre et à un agent surfactant.

3. Procédé selon la revendication 1 ou la revendication 2, caractérisé en ce que le milieu INC comprend un sérum ou un plasma dépourvu d'hormones hypophysaires, obtenu par hypophysectomie d'un animal et un tampon à pH neutre associé à un agent surfactant.

4. Procédé selon la revendication 3, caractérisé en ce que le milieu INC comprend un sérum ou un plasma de bélier hypophysectomisé et un tampon à pH neutre, associé à un agent surfactant.

5. Procédé selon la revendication 2, caractérisé en ce que le plasma ou sérum dépourvu de l'hormone à doser et le tampon sont dans un rapport 1:1.

6. Procédé selon l'une quelconque des revendications 1 à 5, caractérisé en ce que le premier anticorps, fixé sur un support solide, saturé en milieu INC et le deuxième anticorps, éventuellement couplé à une enzyme appropriée, préincubé dans un milieu INC, sont mis en contact avec le fluide biologique à tester, après quoi l'activité enzymatique liée à la phase solide et/ou libre est révélée par tout moyen approprié.

7. Procédé selon la revendication 6, caractérisé en ce que lorsque le deuxième anticorps préincubé dans ledit milieu

EP 0 533 719 B1

INC n'est pas couplé à une enzyme, la révélation de la réaction est alors réalisée par l'introduction d'un troisième anticorps couplé à une enzyme appropriée, préincubé dans ledit milieu INC et se liant spécifiquement au deuxième anticorps.

8. Procédé selon la revendication 6 ou la revendication 7, caractérisé en ce que les premier et deuxième anticorps sont avantageusement choisis dans le groupe qui comprend les anticorps monoclonaux anti-FSH, anti-LH, anti-TSH, anti-GH, anti-ACTH, anti-prolactine, anti-ocytocine, anti-ADH, anti-MSH, et les anticorps polyclonaux anti-FSH, anti-LH, anti-TSH, anti-GH, anti-ACTH, anti-prolactine, anti-ocytocine, anti-ADH et anti-MSH.

9. Procédé selon la revendication 8, caractérisé en ce que les anticorps polyclonaux anti-LH sont notamment obtenus par immunisation d'un animal approprié à l'aide d'un antigène comprenant un mélange équimolaire de différentes fractions purifiées de LH ovine répertoriées 1051, 1055, 1072, 1063, 1085 et 1086, puis par purification de l'immunsérum obtenu par chromatographie d'échange d'ions ou par chromatographie d'affinité, lesquels anticorps polyclonaux présentent un pourcentage de réactions croisées avec les autres hormones glycoprotéiques telles que la FSH, inférieur à 4 %, c'est-à-dire une spécificité vis-à-vis de la LH, en ce qu'ils présentent une polyspécificité de reconnaissance de la LH de nombreuses espèces animales et notamment au moins des espèces ovines, bovines, caprines, porcines, canines, camelines, murines ainsi que les cervidés et en ce qu'ils présentent une affinité vis-à-vis de la LH ovine de l'ordre du $10^9 M^{-1}$, respectivement dans deux espèces animales différentes.

10. Procédé selon l'une quelconque des revendications 6 à 9, caractérisé en ce que les premier et deuxième anticorps sont avantageusement choisis dans le groupe qui comprend des anticorps polyclonaux de Lapin anti-LH ovine et des anticorps polyclonaux de Cheval anti-LH ovine et le troisième anticorps est avantageusement choisi dans le groupe qui comprend les anti-IgG de Cheval et les anti-IgG de Lapin couplés à une enzyme appropriée.

11. Procédé selon l'une quelconque des revendications 6 à 10, caractérisé en ce que le premier anticorps est un anticorps polyclonal de Lapin anti-LH ovine, le deuxième anticorps est un anticorps polyclonal de Cheval anti-LH ovine et le troisième anticorps est un anti-IgG de Cheval couplé à une enzyme appropriée et notamment à une peroxydase ou à une β-galactosidase.

12. Réactif pour la mise en oeuvre du procédé de détection et/ou de dosage immunologique d'hormones chez l'animal y compris l'homme, selon l'une quelconque des revendications 1 à 11, caractérisé en ce qu'il comprend un anticorps anti-hormone propre à être utilisé comme deuxième et/ou troisième anticorps dans ledit procédé, lequel anticorps est préincubé dans un milieu INC.

13. Réactif pour la mise en oeuvre du procédé de détection et/ou de dosage immunologique d'hormones chez l'animal y compris l'homme, selon l'une quelconque des revendications 1 à 11, caractérisé en ce qu'il comprend un anticorps anti-hormone propre à être utilisé comme premier anticorps dans ledit procédé, lequel premier anticorps est fixé sur un support solide, saturé en milieu INC.

14. Trousse de détection et/ou de diagnostic d'hormones hypophysaires (la FSH, la LH, la TSH, la GH, l'ACTH, la prolactine, l'ocytocine, l'ADH, la MSH) chez l'homme ou l'animal, ou kit pour la mise en oeuvre du test selon l'une quelconque des revendications 1 à 11, caractérisé en ce qu'il comprend outre des quantités utiles de tampons pour la mise en oeuvre de ladite détection :

- un premier anticorps, spécifique de l'hormone à doser, choisi parmi les anticorps anti-hormone à doser, fixé sur un support solide, saturé en milieu INC selon la revendication 13 ;
- un deuxième anticorps, spécifique de l'hormone à doser, choisi parmi les anticorps anti-hormone à doser, couplé à une enzyme appropriée et préincubé dans ledit milieu INC, selon la revendication 12 ;
- un substrat de révélation de l'enzyme et
- un milieu INC.

15. Trousse selon la revendication 14, caractérisée en ce que lorsqu'elle est mise en oeuvre pour le dosage de la LH, elle comprend :

- un premier anticorps polyclonal, fixé sur un support solide, saturé en milieu INC, choisi parmi les anticorps polyclonaux de Lapin anti-LH ovine et les anticorps polyclonaux de Cheval anti-LH ovine ;
- un conjugué enzyme-anticorps, dans lequel l'anticorps est choisi dans le groupe qui comprend les anticorps polyclonaux de Lapin anti-LH ovine, les anticorps polyclonaux de Cheval anti-LH ovine, lesquels conjugués

EP 0 533 719 B1

sont préincubés dans un milieu INC ;
- un substrat de révélation de l'enzyme ;
- un capillaire de prélèvement ; et
- un milieu INC.

**16.** Trousse selon la revendication 15, caractérisée en ce que l'enzyme est choisie dans le groupe qui comprend les peroxydases et la β-galactosidase.

**17.** Trousse selon la revendication 14, caractérisée en ce qu'elle comprend :

- une série de supports solides convenables revêtus d'un premier anticorps choisi parmi les anticorps anti-hormone à doser, lesquels supports solides sont saturés en milieu INC ;
- un deuxième anticorps choisi dans le groupe qui comprend les anticorps anti-FSH, anti-LH, anti-TSH, anti-GH, anti-ACTH, anti-prolactine, anti-ocytocine et anti-ADH, différents du premier anticorps, préincubés dans un milieu INC ;
- un conjugué peroxydase-anti-IgG, préincubé dans un milieu INC ;
- un substrat de révélation de la peroxydase ; et
- un milieu INC.

**18.** Trousse selon la revendication 17, caractérisée en ce qu'elle comprend :

- une série de supports solides revêtus d'un premier anticorps choisi dans le groupe qui comprend les anticorps polyclonaux de Lapin anti-LH ovine, lesquels supports solides sont saturés en milieu INC ;
- un deuxième anticorps choisi dans le groupe qui comprend les anticorps polyclonaux de Cheval anti-LH ;
- un conjugué peroxydase-anti-IgG de Cheval préincubé dans un milieu INC ;
- un substrat de révélation de la peroxydase ;
- un capillaire de prélèvement ; et
- un milieu INC.

**19.** Trousse selon la revendication 17, caractérisée en ce qu'elle comprend :

- une série de supports solides revêtus d'un premier anticorps choisi dans le groupe qui comprend les anticorps polyclonaux de Lapin anti-LH ovine, lesquels supports solides sont saturés en milieu INC ;
- un conjugué β-galactosidase-anticorps polyclonaux de Cheval anti-LH ovine préincubé dans un milieu INC ;
- un substrat de révélation de la β-galactosidase ;
- un capillaire de prélèvement ; et
- un milieu INC.

**Revendications pour les Etats contractants suivants : ES, GR**

**1.** Procédé de détection et/ou de dosage immunologique d'hormones hypophysaires humaines ou animales, dans des milieux de culture ou dans des fluides biologiques, mettant en oeuvre au moins deux anticorps, spécifiques de l'hormone à doser, lequel procédé est caractérisé en ce que pour augmenter la sensibilité et la spécificité de ladite détection et/ou dudit dosage, lesdits anticorps sont, -préalablement à leur mise en oeuvre dans ladite détection ou ledit dosage-, préincubés dans un milieu contenant du plasma ou du sérum dépourvu de l'hormone à détecter et/ou à doser, obtenu par hypophysectomie (milieu INC).

**2.** Procédé selon la revendication 1, caractérisé en ce que ledit milieu INC est associé à un tampon à pH neutre et à un agent surfactant.

**3.** Procédé selon la revendication 1 ou la revendication 2, caractérisé en ce que le milieu INC comprend un sérum ou un plasma dépourvu d'hormones hypophysaires, obtenu par hypophysectomie d'un animal et un tampon à pH neutre associé à un agent surfactant.

**4.** Procédé selon la revendication 3, caractérisé en ce que le milieu INC comprend un sérum ou un plasma de bélier hypophysectomisé et un tampon à pH neutre, associé à un agent surfactant.

27

**5.** Procédé selon la revendication 2, caractérisé en ce que le plasma ou sérum dépourvu de l'hormone à doser et le tampon sont dans un rapport 1:1.

**6.** Procédé selon l'une quelconque des revendications 1 à 5, caractérisé en ce que le premier anticorps, fixé sur un support solide, saturé en milieu INC et le deuxième anticorps, éventuellement couplé à une enzyme appropriée, préincubé dans un milieu INC, sont mis en contact avec le fluide biologique à tester, après quoi l'activité enzymatique liée à la phase solide et/ou libre est révélée par tout moyen approprié.

**7.** Procédé selon la revendication 6, caractérisé en ce que lorsque le deuxième anticorps préincubé dans ledit milieu INC n'est pas couplé à une enzyme, la révélation de la réaction est alors réalisée par l'introduction d'un troisième anticorps couplé à une enzyme appropriée, préincubé dans ledit milieu INC et se liant spécifiquement au deuxième anticorps.

**8.** Procédé selon la revendication 6 ou la revendication 7, caractérisé en ce que les premier et deuxième anticorps sont avantageusement choisis dans le groupe qui comprend les anticorps monoclonaux anti-FSH, anti-LH, anti-TSH, anti-GH, anti-ACTH, anti-prolactine, anti-ocytocine, anti-ADH, anti-MSH, et les anticorps polyclonaux anti-FSH, anti-LH, anti-TSH, anti-GH, anti-ACTH, anti-prolactine, anti-ocytocine, anti-ADH et anti-MSH.

**9.** Procédé selon la revendication 8, caractérisé en ce que les anticorps polyclonaux anti-LH sont notamment obtenus par immunisation d'un animal approprié à l'aide d'un antigène comprenant un mélange équimolaire de différentes fractions purifiées de LH ovine répertoriées 1051, 1055, 1072, 1063, 1085 et 1086, puis par purification de l'immunsérum obtenu par chromatographie d'échange d'ions ou par chromatographie d'affinité, lesquels anticorps polyclonaux présentent un pourcentage de réactions croisées avec les autres hormones glycoprotéiques telles que la FSH, inférieur à 4 %, c'est-à-dire une spécificité vis-à-vis de la LH, en ce qu'ils présentent une polyspécificité de reconnaissance de la LH de nombreuses espèces animales et notamment au moins des espèces ovines, bovines, caprines, porcines, canines, camelines, murines ainsi que les cervidés et en ce qu'ils présentent une affinité vis-à-vis de la LH ovine de l'ordre du $10^9 M^{-1}$, respectivement dans deux espèces animales différentes.

**10.** Procédé selon l'une quelconque des revendications 6 à 9, caractérisé en ce que les premier et deuxième anticorps sont avantageusement choisis dans le groupe qui comprend des anticorps polyclonaux de Lapin anti-LH ovine et des anticorps polyclonaux de Cheval anti-LH ovine et le troisième anticorps est avantageusement choisi dans le groupe qui comprend les anti-IgG de Cheval et les anti-IgG de Lapin couplés à une enzyme appropriée.

**11.** Procédé selon l'une quelconque des revendications 6 à 10, caractérisé en ce que le premier anticorps est un anticorps polyclonal de Lapin anti-LH ovine, le deuxième anticorps est un anticorps polyclonal de Cheval anti-LH ovine et le troisième anticorps est un anti-IgG de Cheval couplé à une enzyme appropriée et notamment à une peroxydase ou à une β-galactosidase.

**12.** Procédé de préparation d'un réactif pour la mise en oeuvre du procédé de détection et/ou de dosage immunologique d'hormones chez l'animal y compris l'homme, selon l'une quelconque des revendications 1 à 11, caractérisé en ce qu'il comprend la préparation d'un anticorps anti-hormone propre à être utilisé comme deuxième et/ou troisième anticorps dans ledit procédé, et la préincubation dudit anticorps dans un milieu INC.

**13.** Procédé de préparation d'un réactif pour la mise en oeuvre du procédé de détection et/ou de dosage immunologique d'hormones chez l'animal y compris l'homme, selon l'une quelconque des revendications 1 à 11, caractérisé en ce qu'il comprend la préparation d'un anticorps anti-hormone propre à être utilisé comme premier anticorps dans ledit procédé et la fixation dudit anticorps sur un support solide, saturé en milieu INC.

**14.** Procédé de préparation d'une trousse de détection et/ou de diagnostic d'hormones hypophysaires (la FSH, la LH, la TSH, la GH, l'ACTH, la prolactine, l'ocytocine, l'ADH, la MSH) chez l'homme ou l'animal, ou kit pour la mise en oeuvre du test selon l'une quelconque des revendications 1 à 11, caractérisé en ce qu'il comprend l'association :

- de quantités utiles de tampons,
- d'un premier anticorps, spécifique de l'hormone à doser, choisi parmi les anticorps anti-hormone à doser, fixé sur un support solide, saturé en milieu INC selon la revendication 13 ;
- d'un deuxième anticorps, spécifique de l'hormone à doser, choisi parmi les anticorps anti-hormone à doser, couplé à une enzyme appropriée et préincubé dans ledit milieu INC, selon la revendication 12 ;
- d'un substrat de révélation de l'enzyme et

- d'un milieu INC.

15. Procédé de préparation selon la revendication 14, caractérisé en ce que pour le dosage de la LH, il comprend l'association :

- d'un premier anticorps polyclonal, fixé sur un support solide, saturé en milieu INC, choisi parmi les anticorps polyclonaux de Lapin anti-LH ovine et les anticorps polyclonaux de Cheval anti-LH ovine ;
- d'un conjugué enzyme-anticorps, dans lequel l'anticorps est choisi dans le groupe qui comprend les anticorps polyclonaux de Lapin anti-LH ovine, les anticorps polyclonaux de Cheval anti-LH ovine, lesquels conjugués sont préincubés dans un milieu INC ;
- d'un substrat de révélation de l'enzyme ;
- d'un capillaire de prélèvement ; et
- d'un milieu INC.

16. Procédé de préparation selon la revendication 15, caractérisé en ce que l'enzyme est choisie dans le groupe qui comprend les peroxydases et la β-galactosidase.

17. Procédé de préparation selon la revendication 14, caractérisé en ce qu'il comprend l'association :

- d'une série de supports solides convenables revêtus d'un premier anticorps choisi parmi les anticorps anti-hormone à doser, lesquels supports solides sont saturés en milieu INC ;
- d'un deuxième anticorps choisi dans le groupe qui comprend les anticorps anti-FSH, anti-LH, anti-TSH, anti-GH, anti-ACTH, anti-prolactine, anti-ocytocine et anti-ADH, différents du premier anticorps, préincubés dans un milieu INC ;
- d'un conjugué peroxydase-anti-IgG, préincubé dans un milieu INC ;
- d'un substrat de révélation de la peroxydase ; et
- d'un milieu INC.

18. Procédé de préparation selon la revendication 17, caractérisé en ce qu'il comprend l'association :

- d'une série de supports solides revêtus d'un premier anticorps choisi dans le groupe qui comprend les anticorps polyclonaux de Lapin anti-LH ovine, lesquels supports solides sont saturés en milieu INC ;
- d'un deuxième anticorps choisi dans le groupe qui comprend les anticorps polyclonaux de Cheval anti-LH ;
- d'un conjugué peroxydase-anti-IgG de Cheval préincubé dans un milieu INC ;
- d'un substrat de révélation de la peroxydase ;
- d'un capillaire de prélèvement ; et
- d'un milieu INC.

19. Procédé de préparation selon la revendication 17, caractérisé en ce qu'il comprend l'association :

- d'une série de supports solides revêtus d'un premier anticorps choisi dans le groupe qui comprend les anticorps polyclonaux de Lapin anti-LH ovine, lesquels supports solides sont saturés en milieu INC ;
- d'un conjugué β-galactosidase-anticorps polyclonaux de Cheval anti-LH ovine préincubé dans un milieu INC ;
- d'un substrat de révélation de la β-galactosidase ;
- d'un capillaire de prélèvement ; et
- d'un milieu INC.

**Patentansprüche**

**Patentansprüche für folgende Vertragsstaaten : AT, BE, CH, LI, DE, DK, FR, GB, IT, LU, NL, SE**

1. Verfahren zur Detektion und/oder quantitativen immunologischen Bestimmung von Hormonen der Hypophyse von Menschen oder Tieren in Kulturmedien oder in biologischen Flüssigkeiten, bei dem mindestens zwei Antikörper eingesetzt werden, die für das quantitativ zu bestimmende Hormon spezifisch sind, dadurch **gekennzeichnet,** daß zur Erhöhung der Sensibilität und der Spezifität der Detektion und/oder der quantitativen Bestimmung die Antikörper vor ihrem Einsatz für die Detektion oder die quantitative Bestimmung in einem Medium vorinkubiert werden, welches Plasma oder Serum, das frei ist von dem zu detektierenden und/oder quantitativ zu bestimmenden Hormon und durch Hypophysektomie erhalten worden ist (INC-Medium), enthält.

2. Verfahren nach Anspruch 1, dadurch **gekennzeichnet,** daß das INC-Medium mit einem pH-neutralen Puffer und einem grenzflächenaktiven Mittel assoziiert wird.

3. Verfahren nach Anspruch 1 oder Anspruch 2, dadurch **gekennzeichnet**, daß das INC-Medium ein von Hypophysehormonen freies und durch Hypophysektomie eines Tieres erhaltenes Serum oder Plasma und einen pH-neutralen Puffer, assoziiert mit einem grenzflächenaktiven Mittel, enthält.

4. Verfahren nach Anspruch 3, dadurch **gekennzeichnet** , daß das INC-Medium ein Serum oder ein Plasma eines hypophysektomisierten Widders und einen pH-neutralen Puffer, assoziiert mit einem grenzflächenaktiven Mittel, enthält.

5. Verfahren nach Anspruch 2, dadurch **gekennzeichnet,** daß das Plasma oder Serum, das frei ist von dem quantitativ zu bestimmenden Hormon, und der Puffer in einem Verhältnis von 1:1 vorliegen.

6. Verfahren nach einem der Ansprüche 1 bis 5, dadurch **gekennzeichnet**, daß der erste Antikörper, fixiert auf einem festen Träger, gesättigt in INC-Medium, und der zweite Antikörper, gegebenenfalls gekuppelt an ein geeignetes Enzym, vorinkubiert in einem INC-Medium, mit der zu untersuchenden biologischen Flüssigkeit in Kontakt gebracht werden und darauf die an die feste Phase gebundene und/oder freie enzymatische Aktivität mit jedem geeigneten Mittel nachgewiesen wird.

7. Verfahren nach Anspruch 6, dadurch **gekennzeichnet,** daß, wenn der zweite in dem INC-Medium vorinkubierte Antikörper nicht an ein Enzym gekuppelt ist, der Nachweis der Reaktion durch Einführung eines dritten Antikörpers erfolgt, der an ein geeignetes Enzym gekuppelt und in dem INC-Medium vorinkubiert ist und der sich spezifisch an den zweiten Antikörper bindet.

8. Verfahren nach Anspruch 6 oder Anspruch 7, dadurch **gekennzeichnet**, daß der erste und der zweite Antikörper vorteilhafterweise aus der Gruppe ausgewählt werden, die die monoclonalen Anti-FSH-, Anti-LH-, Anti-TSH-, Anti-GH-, Anti-ACTH-, Anti-Prolactin-, Anti-Ocytocin-, Anti-ADH-, Anti-MSH-Antikörper und die polyclonalen Anti-FSH-, Anti-LH-, Anti-TSH-, Anti-GH-, Anti-ACTH-, Anti-Prolactin-, Anti-Ocytocin-, Anti-ADH- und Anti-MSH-Antikörper umfaßt.

9. Verfahren nach Anspruch 8, dadurch **gekennzeichnet,** daß die polyclonalen Anti-LH-Antikörper vor allem erhalten werden durch Immunisierung eines geeigneten Tieres mit Hilfe eines Antigens, das ein äquimolares Gemisch verschiedener gereinigter Fraktionen von Schaf-LH, registriert unter der Nr. 1051, 1055, 1072, 1063, 1085 und 1086, umfaßt und anschließend durch Reinigung des durch Ionenaustauschchromatographie oder durch Affinitätschromatographie erhaltenen Immunserums, wobei die polyclonalen Antikörper einen Prozentsatz an Kreuzreaktionen mit den anderen Glykoprotein-Hormonen, wie FSH, von weniger als 4%, d.h. eine Spezifität gegenüber LH aufweisen, daß sie eine Polyspezifität der Erkennung von LH von zahlreichen Tierarten, insbesondere mindestens der Arten Schafe, Rinder, Ziegen, Schweine, Hunde, Maus sowie Hirsche aufweisen und eine Affinität gegenüber Schaf-LH in der Größenordnung von $10^9M^{-1}$ jeweils in zwei unterschiedlichen Tierarten aufweisen.

10. Verfahren nach einem der Ansprüche 6 bis 9, dadurch **gekennzeichnet**, daß der erste und der zweite Antikörper vorteilhafterweise aus der Gruppe ausgewählt werden, die polyclonale Kaninchen-Anti-LH-Schaf-Antikörper und polyclonale Pferd-Anti-LH-Schaf-Antikörper umfaßt, und daß der dritte Antikörper vorteilhafterweise aus der Gruppe ausgewählt wird, die die Pferd-Anti-IgG und die Kaninchen-Anti-IgG, gekuppelt an ein geeignetes Enzym, umfaßt.

11. Verfahren nach einem der Ansprüche 6 bis 10, dadurch **gekennzeichnet,** daß der erste Antikörper ein polyclonaler Kaninchen-Anti-LH-Schaf-Antikörper ist, der zweite Antikörper ein polyclonaler Pferd-Anti-LH-Schaf-Antikörper ist und der dritte Antikörper ein Pferd-Anti-IgG, gekuppelt an ein geeignetes Enzym und insbesondere an eine Peroxidase oder an eine β-Galactosidase, ist.

12. Reagens zur Durchführung des Verfahrens der Detektion und/oder der quantitativen immunologischen Bestimmung von Hormonen beim Tier einschließlich des Menschen nach einem der Ansprüche 1 bis 11, dadurch **gekennzeichnet**, daß es einen Anti-Hormon-Antikörper enthält, der als zweiter und/oder dritter Antikörper in dem genannten Verfahren verwendet werden kann, und daß der Antikörper in einem INC-Medium vorinkubiert wird.

13. Reagens für die Durchführung des Verfahrens der Detektion und/oder der quantitativen immunologischen Bestimmung von Hormonen beim Tier einschließlich des Menschen nach einem der Ansprüche 1 bis 11, dadurch **gekennzeichnet,** daß es einen Anti-Hormon-Antikörper enthält, der als erster Antikörper in dem Verfahren verwendet werden kann, wobei der erste Antikörper auf einem festen Träger, gesättigt in INC-Medium, fixiert ist.

14. Besteck für die Detektion und/oder Diagnose von Hypophysehormonen (FSH, LH, TSH, GH, ACTH, Prolactin, Ocytocin, ADH, MSH) bei Menschen oder beim Tier oder Kit für die Durchführung des Tests gemäß einem der Ansprüche 1 bis 11, dadurch **gekennzeichnet,** daß es zusätzlich zu den nützlichen Mengen Puffer für die Ausführung der Detektion umfaßt:

- einen ersten für das quantitativ zu bestimmende Hormon spezifischen Antikörper, ausgewählt aus den Antiquantitativ zu bestimmendes Hormon-Antikörpern, fixiert auf einem festen Träger, gesättigt in INC-Medium, nach Anspruch 13;
- einen zweiten für das quantitativ zu bestimmende Hormon spezifischen Antikörper, ausgewählt aus den Antispezifisch zu bestimmendes Hormon-Antikörpern, gekuppelt an ein geeignetes Enzym und vorinkubiert in dem INC-Medium gemäß Anspruch 12;
- ein Substrat für den Nachweis des Enzyms und
- ein INC-Medium.

15. Besteck nach Anspruch 14, dadurch **gekennzeichnet,** daß es, wenn für die quantitative Bestimmung von LH eingesetzt, umfaßt:

- einen ersten polyclonalen Antikörper, fixiert auf einem festen Träger, gesättigt in INC-Medium, ausgewählt unter den polyclonalen Kaninchen-Anti-LH-Schaf-Antikörpern und den polyclonalen Pferd-Anti-LH-Schaf-Antikörpern;
- ein Enzym-Antikörperkonjugat, in dem der Antikörper ausgewählt ist aus der Gruppe, die die polyclonalen Kaninchen-Anti-LH-Schaf-Antikörper und die polyclonalen Pferd-Anti-LH-Schaf-Antikörper umfaßt, wobei die Konjugate in einem INC-Medium vorinkubiert worden sind;
- ein Substrat für den Nachweis des Enzyms;
- eine Entnahmekapillare; und
- ein INC-Medium.

16. Besteck nach Anspruch 15, dadurch **gekennzeichnet,** daß das Enzym aus der Gruppe, umfassend die Peroxidasen und β-Galactosidase, ausgewählt ist.

17. Besteck nach Anspruch 14, dadurch **gekennzeichnet,** daß es umfaßt:

- eine Reihe von geeigneten festen Trägern, überzogen mit einem ersten Antikörper, ausgewählt unter den Antiquantitativ zu bestimmendes Hormon-Antikörpern, wobei die festen Träger in INC-Medium gesättigt sind;
- einen zweiten Antikörper, ausgewählt aus der Gruppe, die die Antikörper Anti-FSH, Anti-LH, Anti-TSH, Anti-GH, Anti-ACTH, Anti-Prolactin, Anti-Ocytocin und Anti-ADH, die von dem ersten Antikörper verschieden sind, vorinkubiert in einem INC-Medium, enthält;
- ein Peroxidase-Anti-IgG-Konjugat, vorinkubiert in einem INC-Medium;
- ein Substrat für den Nachweis der Peroxidase; und
- ein INC-Medium.

18. Besteck nach Anspruch 17, dadurch **gekennzeichnet,** daß es umfaßt:

- eine Reihe von festen Trägern, überzogen mit einem ersten Antikörper, ausgewählt aus der Gruppe, umfassend die polyclonalen Kaninchen-Anti-LH-Schaf-Antikörper, wobei die festen Träger in INC-Medium gesättigt sind;
- einen zweiten Antikörper, ausgewählt aus der Gruppe, umfassend die polyclonalen Pferd-Anti-LH-Antikörper;
- ein Peroxidase-Pferd-Anti-IgG-Konjugat, vorinkubiert in einem INC-Medium;
- ein Substrat für den Nachweis der Peroxidase;
- eine Entnahmekapillare; und
- ein INC-Medium.

**19.** Besteck nach Anspruch 17, dadurch **gekennzeichnet,** daß es umfaßt:

- eine Reihe von festen Trägern, überzogen mit einem ersten Antikörper, ausgewählt aus der Gruppe, umfassend die polyclonalen Antikörper von Kaninchen-Anti-LH-Schaf, wobei die festen Träger in INC-Medium gesättigt sind;
- ein Konjugat β-Galactosidase-polyclonale Pferd-Anti-LH-Schaf-Antikörper, vorinkubiert in einem INC-Medium;
- ein Substrat für den Nachweis der β-Galactosidase;
- eine Entnahmekapillare; und
- ein INC-Medium.

**Patentansprüche für folgende Vertragsstaaten : ES, GR**

**1.** Verfahren zur Detektion und/oder quantitativen immunologischen Bestimmung von Hormonen der Hypophyse von Menschen oder Tieren in Kulturmedien oder in biologischen Flüssigkeiten, bei dem mindestens zwei Antikörper eingesetzt werden, die für das quantitativ zu bestimmende Hormon spezifisch sind, dadurch **gekennzeichnet,** daß zur Erhöhung der Sensibilität und der Spezifität der Detektion und/oder der quantitativen Bestimmung die Antikörper vor ihrem Einsatz für die Detektion oder die quantitative Bestimmung in einem Medium vorinkubiert werden, welches Plasma oder Serum, das frei ist von dem zu detektierenden und/oder quantitativ zu bestimmenden Hormon und durch Hypophysektomie erhalten worden ist (INC-Medium), enthält.

**2.** Verfahren nach Anspruch 1, dadurch **gekennzeichnet,** daß das INC-Medium mit einem pH-neutralen Puffer und einem grenzflächenaktiven Mittel assoziiert wird.

**3.** Verfahren nach Anspruch 1 oder Anspruch 2, dadurch **gekennzeichnet**, daß das INC-Medium ein von Hypophysehormonen freies und durch Hypophysektomie eines Tieres erhaltenes Serum oder Plasma und einen pH-neutralen Puffer, assoziiert mit einem grenzflächenaktiven Mittel, enthält.

**4.** Verfahren nach Anspruch 3, dadurch **gekennzeichnet,** daß das INC-Medium ein Serum oder ein Plasma eines hypophysektomisierten Widders und einen pH-neutralen Puffer, assoziiert mit einem grenzflächenaktiven Mittel, enthält.

**5.** Verfahren nach Anspruch 2, dadurch **gekennzeichnet,** daß das Plasma oder Serum, das frei ist von dem quantitativ zu bestimmenden Hormon, und der Puffer in einem Verhältnis von 1:1 vorliegen.

**6.** Verfahren nach einem der Ansprüche 1 bis 5, dadurch **gekennzeichnet**, daß der erste Antikörper, fixiert auf einem festen Träger, gesättigt in INC-Medium, und der zweite Antikörper, gegebenenfalls gekuppelt an ein geeignetes Enzym, vorinkubiert in einem INC-Medium, mit der zu untersuchenden biologischen Flüssigkeit in Kontakt gebracht werden und darauf die an die feste Phase gebundene und/oder freie enzymatische Aktivität mit jedem geeigneten Mittel nachgewiesen wird.

**7.** Verfahren nach Anspruch 6, dadurch **gekennzeichnet,** daß, wenn der zweite in dem INC-Medium vorinkubierte Antikörper nicht an ein Enzym gekuppelt ist, der Nachweis der Reaktion durch Einführung eines dritten Antikörpers erfolgt, der an ein geeignetes Enzym gekuppelt und in dem INC-Medium vorinkubiert ist und der sich spezifisch an den zweiten Antikörper bindet.

**8.** Verfahren nach Anspruch 6 oder Anspruch 7, dadurch **gekennzeichnet,** daß der erste und der zweite Antikörper vorteilhafterweise aus der Gruppe ausgewählt werden, die die monoclonalen Anti-FSH-, Anti-LH-, Anti-TSH-, Anti-GH-, Anti-ACTH-, Anti-Prolactin-, Anti-Ocytocin-, Anti-ADH-, Anti-MSH-Antikörper und die polyclonalen Anti-FSH-, Anti-LH-, Anti-TSH-, Anti-GH-, Anti-ACTH-, Anti-Prolactin-, Anti-Ocytocin-, Anti-ADH- und Anti-MSH-Antikörper umfaßt.

**9.** Verfahren nach Anspruch 8, dadurch **gekennzeichnet,** daß die polyclonalen Anti-LH-Antikörper vor allem erhalten werden durch Immunisierung eines geeigneten Tieres mit Hilfe eines Antigens, das ein äquimolares Gemisch verschiedener gereinigter Fraktionen von Schaf-LH, registriert unter der Nr. 1051, 1055, 1072, 1063, 1085 und 1086, umfaßt und anschließend durch Reinigung des durch Ionenaustauschchromatographie oder durch Affinitätschromatographie erhaltenen Immunserums, wobei die polyclonalen Antikörper einen Prozentsatz an Kreuzreaktionen mit den anderen Glykoprotein-Hormonen, wie FSH, von weniger als 4%, d.h. eine Spezifität gegenüber LH aufweisen, daß sie eine Polyspezifität der Erkennung von LH von zahlreichen Tierarten, insbesondere mindestens der Arten Schafe, Rinder, Ziegen, Schweine, Hunde, Maus sowie Hirsche aufweisen und eine Affinität gegenüber Schaf-LH in der Größenordnung von $10^9 M^{-1}$ jeweils in zwei unterschiedlichen Tierarten aufweisen.

10. Verfahren nach einem der Ansprüche 6 bis 9, dadurch **gekennzeichnet**, daß der erste und der zweite Antikörper vorteilhafterweise aus der Gruppe ausgewählt werden, die polyclonale Kaninchen-Anti-LH-Schaf-Antikörper und polyclonale Pferd-Anti-LH-Schaf-Antikörper umfaßt, und daß der dritte Antikörper vorteilhafterweise aus der Gruppe ausgewählt wird, die die Pferd-Anti-IgG und die Kaninchen-Anti-IgG, gekuppelt an ein geeignetes Enzym, umfaßt.

11. Verfahren nach einem der Ansprüche 6 bis 10, dadurch **gekennzeichnet**, daß der erste Antikörper ein polyclonaler Kaninchen-Anti-LH-Schaf-Antikörper ist, der zweite Antikörper ein polyclonaler Pferd-Anti-LH-Schaf-Antikörper ist und der dritte Antikörper ein Pferd-Anti-IgG, gekuppelt an ein geeignetes Enzym und insbesondere an eine Peroxidase oder an eine β-Galactosidase, ist.

12. Verfahren zur Herstellung eines Reagens für die Durchführung des Verfahrens zur Detektion und/oder quantitativen immunologischen Bestimmung von Hormonen beim Tier einschließlich des Menschen nach einem der Ansprüche 1 bis 11, dadurch **gekennzeichnet**, daß es die Herstellung eines Anti-Hormon-Antikörpers umfaßt, der als zweiter und/oder dritter Antikörper in dem Verfahren eingesetzt werden kann, sowie die Vorinkubation des Antikörpers in einem INC-Medium.

13. Verfahren zur Herstellung eines Reagens für die Durchführung des Verfahrens der Detektion und/oder quantitativen immunologischen Bestimmung von Hormonen beim Tier einschließlich des Menschen nach einem der Ansprüche 1 bis 11, dadurch **gekennzeichnet**, daß es die Herstellung eines Anti-Hormon-Antikörpers, der als erster Antikörper in dem Verfahren verwendet werden kann, und die Fixierung des Antikörpers auf einem festen Träger, gesättigt in INC-Medium, umfaßt.

14. Verfahren zur Herstellung eines Bestecks für die Detektion und/oder die Diagnose von Hypophysehormonen (FSH, LH, TSH, GH, ACTH, Prolactin, Ocytocin, ADH, MSH) beim Menschen oder beim Tier oder eines Kit für die Durchführung des Tests nach einem der Ansprüche 1 bis 11, dadurch **gekennzeichnet,** daß es die Assoziation umfaßt von:

- nützliche Mengen an Puffern;
- einen ersten für das quantitativ zu bestimmende Hormon spezifischen Antikörper, ausgewählt aus den Antiquantitativ zu bestimmendes Hormon-Antikörpern, fixiert auf einem festen Träger, gesättigt in INC-Medium nach Anspruch 13;
- einen zweiten für das quantitativ zu bestimmende Hormon spezifischen Antikörper, ausgewählt aus den Antiquantitativ zu bestimmendes Hormon-Antikörpern, gekuppelt an ein geeignetes Enzym und vorinkubiert in dem INC-Medium, nach Anspruch 12;
- ein Substrat für den Nachweis des Enzyms; und
- ein INC-Medium.

15. Verfahren zur Herstellung nach Anspruch 14, dadurch **gekennzeichnet**, daß es für die quantitative Bestimmung von LH die folgende Assoziation umfaßt:

- einen ersten polyclonalen Antikörper, fixiert auf einem festen Träger, gesättigt in INC-Medium, ausgewählt unter den polyclonalen Kaninchen-Anti-LH-Schaf-Antikörpern und den polyclonalen Pferd-Anti-LH-Schaf-Antikörpern;
- ein Enzym-Antikörperkonjugat, in dem der Antikörper ausgewählt ist aus der Gruppe, die die polyclonalen Kaninchen-Anti-LH-Schaf-Antikörper und die polyclonalen Pferd-Anti-LH-Schaf-Antikörper umfaßt, wobei die Konjugate in einem INC-Medium vorinkubiert worden sind;
- ein Substrat für den Nachweis des Enzyms;
- eine Entnahmekapillare; und
- ein INC-Medium.

16. Verfahren zur Herstellung nach Anspruch 15, dadurch **gekennzeichnet**, daß das Enzym aus der Gruppe, umfassend die Peroxidasen und die ß-Galactosidase, ausgewählt wird.

**17.** Verfahren zur Herstellung nach Anspruch 14, dadurch **gekennzeichnet,** daß es die Assoziation umfaßt aus:

- eine Reihe von geeigneten festen Trägern, überzogen mit einem ersten Antikörper, ausgewählt unter den Antiquantitativ zu bestimmendes Hormon-Antikörpern, wobei die festen Träger in INC-Medium gesättigt sind;
- einen zweiten Antikörper, ausgewählt aus der Gruppe, die die Antikörper Anti-FSH, Anti-LH, Anti-TSH, Anti-GH, Anti-ACTH, Anti-Prolactin, Anti-Ocytocin und Anti-ADH, die von dem ersten Antikörper verschieden sind, vorinkubiert in einem INC-Medium, enthält;
- ein Peroxidase-Anti-IgG-Konjugat, vorinkubiert in einem INC-Medium;
- ein Substrat für den Nachweis der Peroxidase; und
- ein INC-Medium.

**18.** Verfahren zur Herstellung nach Anspruch 17, dadurch **gekennzeichnet**, daß es folgende Assoziation umfaßt:

- eine Reihe von festen Trägern, überzogen mit einem ersten Antikörper, ausgewählt aus der Gruppe, umfassend die polyclonalen Kaninchen-Anti-LH-Schaf-Antikörper, wobei die festen Träger in INC-Medium gesättigt sind;
- einen zweiten Antikörper, ausgewählt aus der Gruppe, umfassend die polyclonalen Pferd-Anti-LH-Antikörper;
- ein Peroxidase-Pferd-Anti-IgG-Konjugat, vorinkubiert in einem INC-Medium;
- ein Substrat für den Nachweis der Peroxidase;
- eine Entnahmekapillare; und
- ein INC-Medium.

**19.** Verfahren zur Herstellung nach Anspruch 17, dadurch **gekennzeichnet**, daß es folgende Assoziation umfaßt:

- eine Reihe von festen Trägern, überzogen mit einem ersten Antikörper, ausgewählt aus der Gruppe, umfassend die polyclonalen Antikörper von Kaninchen-Anti-LH-Schaf, wobei die festen Träger in INC-Medium gesättigt sind;
- ein Konjugat β-Galactosidase-polyclonale Pferd-Anti-LH-Schaf-Antikörper, vorinkubiert in einem INC-Medium;
- ein Substrat für den Nachweis der β-Galactosidase;
- eine Entnahmekapillare; und
- ein INC-Medium.

## Claims

**Claims for the following Contracting States : AT, BE, CH, LI, DE, DK, FR, GB, IT, LU, NL, SE**

**1.** Process of immunological detection and/or assay of human or animal hypophysial hormones in culture media or in biological fluids, employing at least two antibodies specific for the hormone to be assayed, which process is characterised in that for increasing the sensitivity and the specificity of the said detection and/or assay, the said antibodies are, -prior to their employment in the said detection or the said assay-, pre-incubated in a medium containing plasma or serum free of the hormone to be detected and/or to be assayed, obtained by hypophysectomy (INC medium).

**2.** Process according to claim 1, characterised in that the said INC medium is combined with a buffer with a neutral pH, and a surfactant agent.

**3.** Process according to claim 1 or claim 2, characterised in that the INC medium comprises a serum or a plasma which is free of hypophysial hormones, obtained by hypophysectomy of an animal, and, optionally, a buffer with a neutral pH associated with a surfactant agent.

**4.** Process according to claim 3, characterised in that the INC medium comprises a serum or a plasma from a hypophysectomised ram and a buffer with a neutral pH, associated with a surfactant agent.

**5.** Process according to claim 2, characterised in that the plasma or serum free of the hormone to be assayed and the buffer are in a ratio of 1:1.

**6.** Process according to any one of claims 1 to 5, characterised in that the first antibody, fixed on a solid support, saturated with INC medium, and the second antibody optionally linked to a suitable enzyme, pre-incubated in an INC medium, are brought into contact with the biological fluid to be tested, after which the enzymatic activity associated with the solid and/or free phase is detected by any suitable means.

**7.** Process according to claim 6, characterised in that, when the second antibody pre-incubated in the said INC medium is not linked to an enzyme, the reaction is then detected by the introduction of a third antibody linked to a suitable enzyme, pre-incubated in the said INC medium and binding specifically to the second antibody.

**8.** Process according to claim 6 or claim 7, characterised in that the first and second antibodies are advantageously chosen from the group which comprises the monoclonal anti-FSH, anti-LH, anti-TSH, anti-GH, anti-ACTH, anti-prolactin, anti-oxytocin, anti-ADH, anti-MSH antibodies and the polyclonal anti-FSH, anti-LH, anti-TSH, anti-GH, anti-ACTH, anti-prolactin, anti-oxytocin, anti-ADH, anti-MSH antibodies.

**9.** Process according to claim 8, characterised in that the polyclonal anti-LH antibodies are obtained in particular by immunising a suitable animal with an antigen comprising an equimolar mixture of various purified fractions of ovine LH, listed 1051, 1055, 1072, 1063, 1085 and 1086, then by purification of the immune serum obtained by ion-exchange chromatography or affinity chromatography, which polyclonal antibodies have a percentage of cross-reactions with the other glycoprotein hormones, such as FSH, of less than 4%, that is to say a specificity with respect to LH, in that they have a polyspecificity of recognition of the LH of numerous animal species and, in particular, at least the ovine, bovine, caprine, porcine, canine, cameline and murine species, as well as the cervidae, and in that they have an affinity with respect to ovine LH of the order of $10^9 M^{-1}$, respectively in two different animal species.

**10.** Process according to any one of claims 6 to 9, characterised in that the first and second antibodies are advantageously chosen from the group which comprises polyclonal rabbit anti-sheep LH antibodies and polyclonal horse anti-sheep LH antibodies, and the third antibody is advantageously chosen from the group which comprises the horse anti-IgG and the rabbit anti-IgG linked to a suitable enzyme.

**11.** Process according to any one of claims 6 to 10, characterised in that the first antibody is a polyclonal rabbit anti-sheep LH antibody, the second antibody is a polyclonal horse anti-sheep LH antibody, and the third antibody is a horse anti-IgG linked to a suitable enzyme and in particular to a peroxidase or to a β-galactosidase.

**12.** Reagent for the implementation of the process for the immunological detection and/or assay of hormones in animals, including man, according to any one of claims 1 to 11, characterised in that it comprises an anti-hormone antibody capable of being used as the second and/or third antibody in the said process, which antibody is pre-incubated in an INC medium.

**13.** Reagent for the implementation of the process for the immunological detection and/or assay of hormones in animals, including man, according to any one of claims 1 to 11, characterised in that it comprises an anti-hormone antibody capable of being used as the first antibody in the said process, which antibody is fixed on a solid support, saturated with INC medium.

**14.** Kit for the detection and/or diagnosis of hypophysial hormones (FSH, LH, TSH, GH, ACTH, prolactin, oxytocin, ADH, MSH), in man or animal, or kit for the implementation of the test according to any one of claims 1 to 11, characterised in that it comprises, in addition to useful quantities of suitable buffers for the implementation of the said detection:

- a first antibody, specific for the hormone to be assayed, chosen from among the appropriate anti-hormone antibodies, fixed on a solid support, saturated with INC medium according to claim 13;
- a second antibody, specific for the hormone to be assayed, chosen from the group which comprises the antibodies to the hormone to be assayed, linked to an appropriate enzyme and pre-incubated in said INC medium, according to claim 12;
- a substrate for detection of the enzyme; and
- an INC medium.

**15.** Kit according to claim 14, characterised in that, when it is used for the assay of LH, it comprises:

- a first polyclonal antibody, fixed on a solid support, saturated with INC medium, chosen from among the polyclonal rabbit anti-sheep LH antibodies and the polyclonal horse anti-sheep LH antibodies;
- an enzyme/antibody conjugate, in which the antibody is chosen from the group which comprises the polyclonal rabbit anti-sheep LH antibodies, the polyclonal horse anti-sheep LH antibodies, which conjugate is pre-incubated in an INC medium;
- a substrate for detection of the enzyme;
- a sampling capillary; and
- an INC medium.

16. Kit according to claim 15, characterised in that the enzyme is chosen from the group which comprises the peroxidases and β-galactosidase.

17. Kit according to claim 14, characterised in that it comprises:

- a series of appropriate solid supports covered with a first antibody chosen from among the antibodies to the hormone to be assayed, which solid supports are saturated with INC medium;
- a second antibody chosen from the group which comprises the antibodies anti-FSH, anti-LH, anti-TSH, anti-GH, anti-ACTH, anti-prolactin, anti-oxytocin, anti-ADH, different from the first antibody, pre-incubated with an INC medium;
- an appropriate peroxidase/anti-IgG conjugate, pre-incubated in an INC medium;
- a substrate for detection of the peroxidase; and
- an INC medium.

18. Kit according to claim 17, characterised in that it comprises:

- a series of appropriate solid supports covered with a first antibody chosen from the group which comprises the polyclonal rabbit anti-sheep LH antibodies, which solid supports are saturated with INC medium;
- a second antibody chosen from the group which comprises the polyclonal horse anti-sheep LH antibodies;
- a peroxidase/horse anti-IgG conjugate, pre-incubated in an INC medium;
- a substrate for detection of the peroxidase;
- a sampling capillary tube; and
- an INC medium.

19. Kit according to claim 17, characterised in that it comprises:

- a series of appropriate solid supports covered with a first antibody chosen from the group which comprises the polyclonal rabbit anti-sheep LH antibodies, which solid supports are saturated with INC medium;
- a conjugate of β-galactosidase and polyclonal horse anti-sheep LH antibodies, pre-incubated in an INC medium, in conformity with the reagent described herein-above;
- a substrate for detection of β-galactosidase;
- a sampling capillary tube; and
- an INC medium.

**Claims for the following Contracting States : ES, GR**

1. Process of immunological detection and/or assay of human or animal hypophysial hormones in culture media or in biological fluids, employing at least two antibodies specific for the hormone to be assayed, which process is characterised in that for increasing the sensitivity and the specificity of the said detection and/or assay, the said antibodies are, -prior to their employment in the said detection or the said assay-, pre-incubated in a medium containing plasma or serum free of the hormone to be detected and/or to be assayed, obtained by hypophysectomy (INC medium).

2. Process according to claim 1, characterised in that the said INC medium is combined with a buffer with a neutral pH, and a surfactant agent.

3. Process according to claim 1 or claim 2, characterised in that the INC medium comprises a serum or a plasma which is free of hypophysial hormones, obtained by hypophysectomy of an animal, and, optionally, a buffer with a neutral pH associated with a surfactant agent.

4. Process according to claim 3, characterised in that the INC medium comprises a serum or a plasma from a hypophysectomised ram and a buffer with a neutral pH, associated with a surfactant agent.

5. Process according to claim 2, characterised in that the plasma or serum free of the hormone to be assayed and the buffer are in a ratio of 1:1.

6. Process according to any one of claims 1 to 5, characterised in that the first antibody, fixed on a solid support, saturated with INC medium, and the second antibody optionally linked to a suitable enzyme, pre-incubated in an INC medium, are brought into contact with the biological fluid to be tested, after which the enzymatic activity associated with the solid and/or free phase is detected by any suitable means.

7. Process according to claim 6, characterised in that, when the second antibody pre-incubated in the said INC medium is not linked to an enzyme, the reaction is then detected by the introduction of a third antibody linked to a suitable enzyme, pre-incubated in the said INC medium and binding specifically to the second antibody.

8. Process according to claim 6 or claim 7, characterised in that the first and second antibodies are advantageously chosen from the group which comprises the monoclonal anti-FSH, anti-LH, anti-TSH, anti-GH, anti-ACTH, anti-prolactin, anti-oxytocin, anti-ADH, anti-MSH antibodies and the polyclonal anti-FSH, anti-LH, anti-TSH, anti-GH, anti-ACTH, anti-prolactin, anti-oxytocin, anti-ADH, anti-MSH antibodies.

9. Process according to claim 8, characterised in that the polyclonal anti-LH antibodies are obtained in particular by immunising a suitable animal with an antigen comprising an equimolar mixture of various purified fractions of ovine LH, listed 1051, 1055, 1072, 1063, 1085 and 1086, then by purification of the immune serum obtained by ion-exchange chromatography or affinity chromatography, which polyclonal antibodies have a percentage of cross-reactions with the other glycoprotein hormones, such as FSH, of less than 4%, that is to say a specificity with respect to LH, in that they have a polyspecificity of recognition of the LH of numerous animal species and, in particular, at least the ovine, bovine, caprine, porcine, canine, cameline and murine species, as well as the cervidae, and in that they have an affinity with respect to ovine LH of the order of $10^9 M^{-1}$, respectively in two different animal species.

10. Process according to any one of claims 6 to 9, characterised in that the first and second antibodies are advantageously chosen from the group which comprises polyclonal rabbit anti-sheep LH antibodies and polyclonal horse anti-sheep LH antibodies, and the third antibody is advantageously chosen from the group which comprises the horse anti-IgG and the rabbit anti-IgG linked to a suitable enzyme.

11. Process according to any one of claims 6 to 10, characterised in that the first antibody is a polyclonal rabbit anti-sheep LH antibody, the second antibody is a polyclonal horse anti-sheep LH antibody, and the third antibody is a horse anti-IgG linked to a suitable enzyme and in particular to a peroxidase or to a β-galactosidase.

12. Process of preparation of a reagent for the implementation of the process for the immunological detection and/or assay of hormones in animals, including man, according to any one of claims 1 to 11, characterised in that it comprises the preparation of an anti-hormone antibody capable of being used as the second and/or third antibody in the said process, which antibody is pre-incubated in an INC medium.

13. Process of preparation of a reagent for the implementation of the process for the immunological detection and/or assay of hormones in animals, including man, according to any one of claims 1 to 11, characterised in that it comprises the preparation of an anti-hormone antibody capable of being used as the first antibody in the said process, which antibody is fixed on a solid support, saturated with INC medium.

14. Process of preparation of a kit for the detection and/or diagnosis of hypophysial hormones (FSH, LH, TSH, GH, ACTH, prolactin, oxytocin, ADH, MSH), in man or animal, or kit for the implementation of the test according to any one of claims 1 to 11, characterised in that it comprises the association of:

- useful quantities of suitable buffers;
- a first antibody, specific for the hormone to be assayed, chosen from among the appropriate anti-hormone antibodies, fixed on a solid support, saturated with INC medium according to claim 13;

- a second antibody, specific for the hormone to be assayed, chosen from the group which comprises the antibodies to the hormone to be assayed, linked to an appropriate enzyme and pre-incubated in said INC medium, according to claim 12;
- a substrate for detection of the enzyme; and
- an INC medium.

15. Process of preparation according to claim 14, characterised in that, when it is used for the assay of LH, it comprises the association of:

- a first polyclonal antibody, fixed on a solid support, saturated with INC medium, chosen from among the polyclonal rabbit anti-sheep LH antibodies and the polyclonal horse anti-sheep LH antibodies;
- an enzyme/antibody conjugate, in which the antibody is chosen from the group which comprises the polyclonal rabbit anti-sheep LH antibodies, the polyclonal horse anti-sheep LH antibodies, which conjugate is pre-incubated in an INC medium;
- a substrate for detection of the enzyme;
- a sampling capillary; and
- an INC medium.

16. Process of preparation according to claim 15, characterised in that the enzyme is chosen from the group which comprises the peroxidases and β-galactosidase.

17. Process of preparation according to claim 14, characterised in that it comprises the association of:

- a series of appropriate solid supports covered with a first antibody chosen from among the antibodies to the hormone to be assayed, which solid supports are saturated with INC medium;
- a second antibody chosen from the group which comprises the antibodies anti-FSH, anti-LH, anti-TSH, anti-GH, anti-ACTH, anti-prolactin, anti-oxytocin, anti-ADH, different from the first antibody, pre-incubated with an INC medium;
- an appropriate peroxidase/anti-IgG conjugate, pre-incubated in an INC medium;
- a substrate for detection of the peroxidase; and
- an INC medium.

18. Process of preparation according to claim 17, characterised in that it comprises the association of:

- a series of appropriate solid supports covered with a first antibody chosen from the group which comprises the polyclonal rabbit anti-sheep LH antibodies, which solid supports are saturated with INC medium;
- a second antibody chosen from the group which comprises the polyclonal horse anti-sheep LH antibodies;
- a peroxidase/horse anti-IgG conjugate, pre-incubated in an INC medium;
- a substrate for detection of the peroxidase;
- a sampling capillary tube; and
- an INC medium.

19. Process of preparation according to claim 17, characterised in that it comprises the association of:

- a series of appropriate solid supports covered with a first antibody chosen from the group which comprises the polyclonal rabbit anti-sheep LH antibodies, which solid supports are saturated with INC medium;
- a conjugate of β-galactosidase and polyclonal horse anti-sheep LH antibodies, pre-incubated in an INC medium, in conformity with the reagent described herein-above;
- a substrate for detection of β-galactosidase;
- a sampling capillary tube; and
- an INC medium.

FIG.1

FIG. 2

EP 0 533 719 B1

FIG. 3

FIG. 4

EP 0 533 719 B1

FIG. 5

FIG. 6

FIG. 7

courbe A  (✳)

Concentration (ng/ml)

courbe B (□)

Concentration (ng/ml)

FIG. 8

EP 0 533 719 B1

BREBIS

↑ 1 . 2 . 3 . 4 . 5 . 6 . 7 . 8 .

Gamme de concentrations

A
B
C
D
E
F
G
H

Pic de LH :

| A | | | | | | | |
|---|---|---|---|---|---|---|---|
| B | G | E | C | C | E | E | |
| C | H | F | D | D | F | F | |
| D | | G | G | E | G | G | G |
| | | H | H | F | H | H | H |
| | | | | H | | | |

FIG. 9

B

FIG. 10

FIG.11

**A**

Gamme de concentrations

| | Vache 1 | Vache 2 | Vache 3 | Vache 4 |
|---|---|---|---|---|
| Pic de LH: | F G | pas de pic | D E F G | C D E |

<u>FIG.12</u>

**B**

FIG. 13

**A**

.2. 3. 4. 5. 6. 7. 8  9.10

Gamme de
concentrations

Bélier 1
pulse de LH en 9

Bélier 2
pulses en 4. 9.10

Bélier 3
pulse en 4

FIG.14

**B**

FIG. 15

FIG. 16

FIG. 17

FIG. 18

FIG. 19

FIG. 20

FIG. 21